(19) Europäisches Patentamt
European Patent Office
Office européen des brevets

(11) Veröffentlichungsnummer: **0 075 167**
**B1**

# (12) EUROPÄISCHE PATENTSCHRIFT

(45) Veröffentlichungstag der Patentschrift: **13.02.85**

(21) Anmeldenummer: **82108184.1**

(22) Anmeldetag: **06.09.82**

(51) Int. Cl.⁴: **C 07 C 103/365**, C 07 C 103/76, C 07 C 125/063, C 07 C 127/19, C 07 C 147/05, C 07 C 147/14, C 07 C 143/68, C 07 C 149/23, C 07 D 231/12, C 07 D 233/58, C 07 D 249/08

(54) **N-(1-Alkenyl)-carbonsäureanilide, Verfahren zu ihrer Herstellung sowie ihre Verwendung als Fungizide.**

(30) Priorität: **18.09.81 DE 3137299**

(43) Veröffentlichungstag der Anmeldung: **30.03.83 Patentblatt 83/13**

(45) Bekanntmachung des Hinweises auf die Patenterteilung: **13.02.85 Patentblatt 85/7**

(84) Benannte Vertragsstaaten: **AT BE CH DE FR GB IT LI NL**

(56) Entgegenhaltungen:
**EP-A-0 018 509**
**EP-A-0 020 859**
**EP-A-0 037 019**
**FR-A-2 416 216**

(73) Patentinhaber: **BAYER AG, Konzernverwaltung RP Patentabteilung, D-5090 Leverkusen 1 Bayerwerk (DE)**

(72) Erfinder: **Thomas, Rudolf, Dr., Dellbusch 269, D-5600 Wuppertal 2 (DE)**
Erfinder: **Brandes, Wilhelm, Dr., Eichendorffstrasse 3, D-5653 Leichlingen 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 075 167 B1**

BUNDESDRUCKEREI BERLIN

**Beschreibung**

Die vorliegende Erfindung betrifft neue N-(1-Alkenyl)-carbonsäureanilide, mehrere Verfahren zu ihrer Herstellung und ihre Verwendung als Fungizide.

Es ist bereits bekanntgeworden, daß Zink-ethylen-1,2-bis-ditiocarbamidat gute fungizide Eigenschaften aufweist (vgl. R. Wegler, Chemie der Pflanzenschutz- und Schädlingsbekämpfungsmittel, Band 2; S. 65, 1970).

Ebenfalls bekannt ist, daß N-Chloracetyl-N-(2,6-dialkylphenyl)-alanin-alkylester, wie z. B. N-Chloracetyl-N-(2,6-dimethylphenyl)-alanin-ethylester, mit gutem Erfolg zur Bekämpfung von pilzlichen Pflanzenkrankheiten eingesetzt werden können (vgl. DE-OS 2 350 944). Die Wirkung der genannten Verbindungen ist jedoch, insbesondere bei niedrigen Aufwandmengen und -konzentrationen, insbesondere auch bei der Bekämpfung von Phytophthora-Arten, nicht immer ganz befriedigend. Weiterhin sind N-Allyl- und N-Allenylacetanilide, wie z. B. 2,6-Dimethyl-N-allylimidazolylacetanilid und 2,6-Dimethyl-N-allenyl-ethoxyacetanilid, und ihre Verwendung als Fungizide bekannt (vgl. EP-A-18 509 und EP-A-20 859).

Es wurden neue N-(1-Alkenyl)-carbonsäureanilide der allgemeinen Formel (I)

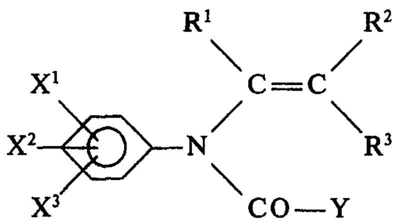

(I)

in welcher

$R^1$ für Wasserstoff und Alkyl steht,
$R^2$ für Wasserstoff, Alkyl, Alkoxy, Alkoxyalkyl, Hydroxyalkyl oder Benzyl steht,
$R^3$ für Wasserstoff, Alkyl, Alkenyl, Alkoxy, Alkoxyalkyl, Alkylcarbonyl, Alkylcarbonylalkyl, Alkoxycarbonyl oder Alkoxycarbonylalkyl steht, oder
$R^1$ und $R^2$ gemeinsam mit der C=C-Doppelbindung für einen gegebenenfalls ein- oder mehrfach durch Alkyl mit 1 bis 4 Kohlenstoffatomen, Halogen und Hydroxy substituierten, einfach oder mehrfach ungesättigten Ring stehen, der auch Heteroatome enthalten kann, oder
$R^2$ und $R^3$ gemeinsam mit dem angrenzenden Kohlenstoffatom für einen gegebenenfalls ein- oder mehrfach durch Alkyl mit 1 bis 4 Kohlenstoffatomen, Halogen und Hydroxy substituierten, gesättigten oder ungesättigten Ring stehen, der auch Heteroatome enthalten kann,
$X^1$, $X^2$ und $X^3$ unabhängig voneinander für Wasserstoff, Alkyl oder Halogen stehen,
Y für Furyl, Tetrahydrofuryl, Thienyl, Tetrahydrothienyl; gegebenenfalls durch Halogen, Alkyl, Alkoxy und Alkylthio mit jeweils 1 bis 4 Kohlenstoffatomen, Halogenalkyl, Halogenalkoxy und Halogenalkylthio mit jeweils 1 bis 4 Kohlenstoff- und 1 bis 5 gleichen oder verschiedenen Halogenatomen, wie Fluor- und Chloratomen, Nitro, Cyano sowie gegebenenfalls durch Halogen und Alkyl mit 1 bis 4 Kohlenstoffatomen substituiertes Phenyl, substituiertes Phenyl; gegebenenfalls durch Alkyl substituiertes Isoxazolyl; 1,2,4-Triazol-1-yl, Imidazol-1-yl und Pyrazol-1-yl, gegebenenfalls durch Cyano oder Thiocyano substituiertes Alkyl, Alkenyl und Alkinyl, gegebenenfalls durch Alkyl mit 1 bis 4 Kohlenstoffatomen, Alkenyl mit 2 bis 4 Kohlenstoffatomen, Halogenalkyl mit 1 bis 4 Kohlenstoff- und bis zu 5 gleichen oder verschiedenen Halogenatomen, Halogenalkenyl mit 2 bis 4 Kohlenstoff- und bis zu 5 gleichen oder verschiedenen Halogenatomen, Halogen, Alkoxy mit 1 bis 4 Kohlenstoffatomen sowie eine zwei- bis fünfgliedrige Methylenbrücke substituiertes Cycloalkyl oder Cycloalkenyl; Dihalogenalkyl; sowie die Gruppierungen

$-CH_2Az$ $-CH_2R^9$ $-CH_2OR^8$ $-CH_2OR^9$ $-CH_2SR^8$ $-CH_2SR^9$

$-CH_2-SO_nR^8$ $-CH_2SO_n-R^9$ $-CH_2OSOR^8$ $-CH_2OSOR^9$ $-CH_2OSO_2R^8$

$-CH_2OSO_2R^9$ $-CH_2OCOR^8$ $-CH_2OCOR^9$ $-OR^8$ $-OR^9$ $-SR^8$

$-SR^9$ $-NR^{10}R^{11}$ $-CH_2-O-$ 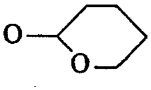

steht, wobei

$R^8$ für gegebenenfalls durch Halogen, Cyano oder Thiocyano substituiertes Alkyl, Alkenyl, Alkinyl und Alkoxyalkyl steht,

$R^9$ für gegebenenfalls durch Halogen, Alkyl, Alkoxy und Alkylthio mit jeweils 1 bis 4 Kohlenstoffatomen, Halogenalkyl, Halogenalkoxy und Halogenalkylthio mit jeweils 1 bis 4 Kohlenstoff- und 1 bis 5 gleichen oder verschiedenen Halogenatomen, wie Fluor- und Chloratomen, Nitro-, Cyano sowie gegebenenfalls durch Halogen und Alkyl mit 1 bis 4 Kohlenstoffatomen substituiertes Phenyl substituiertes Phenyl steht,

$R^{10}$ und $R^{11}$ gleich oder verschieden sind und für Wasserstoff, Alkyl, Alkenyl oder gegebenenfalls durch Halogen, Alkyl, Alkoxy und Alkylthio mit jeweils 1 bis 4 Kohlenstoffatomen, Halogenalkyl, Halogenalkoxy und Halogenalkylthio mit jeweils 1 bis 4 Kohlenstoff- und 1 bis 5 gleichen oder verschiedenen Halogenatomen, wie Fluor- und Chloratomen, Nitro, Cyano sowie gegebenenfalls durch Halogen und Alkyl mit 1 bis 4 Kohlenstoffatomen substituiertes Phenyl substituiertes Phenyl stehen,

Az für Pyrazol-1-yl, 1,2,4-Triazol-1-yl und Imidazol-1-yl steht,

n für 1 oder 2 steht,

sowie, wenn

Y für Az bzw. für $-CH_2-Az$ steht, deren physiologisch verträglichen Säureadditionssalze und Metallsalzkomplexe.

Weiterhin wurde gefunden, daß man die neuen N-(1-Alkenyl)-carbonsäureanilide der Formel (I)

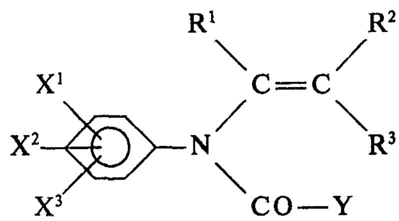

(I)

in welcher

$R^1$ für Wasserstoff und Alkyl steht,

$R^2$ für Wasserstoff, Alkyl, Alkoxy, Alkoxyalkyl, Hydroxyalkyl oder Benzyl steht,

$R^3$ für Wasserstoff, Alkyl, Alkenyl, Alkoxy, Alkoxyalkyl, Alkylcarbonyl, Alkylcarbonylalkyl, Alkoxycarbonyl oder Alkoxycarbonylalkyl steht, oder

$R^1$ und $R^2$ gemeinsam mit der C=C-Doppelbindung für einen gegebenenfalls ein- oder mehrfach durch Alkyl mit 1 bis 4 Kohlenstoffatomen, Halogen und Hydroxy substituierten, einfach oder mehrfach ungesättigten Ring stehen, der auch Heteroatome enthalten kann, oder

$R^2$ und $R^3$ gemeinsam mit dem angrenzenden Kohlenstoffatom für einen gegebenenfalls ein- oder mehrfach durch Alkyl mit 1 bis 4 Kohlenstoffatomen, Halogen und Hydroxy substituierten, gesättigten oder ungesättigten Ring stehen, der auch Heteroatome enthalten kann,

$X^1$, $X^2$ und $X^3$ unabhängig voneinander für Wasserstoff, Alkyl oder Halogen stehen,

Y für Furyl, Tetrahydrofuryl, Thienyl, Tetrahydrothienyl; gegebenenfalls durch Halogen, Alkyl, Alkoxy und Alkylthio mit jeweils 1 bis 4 Kohlenstoffatomen, Halogenalkyl, Halogenalkoxy und Halogenalkylthio mit jeweils 1 bis 4 Kohlenstoff- und 1 bis 5 gleichen oder verschiedenen Halogenatomen, wie Fluor- und Chloratomen, Nitro, Cyano sowie gegebenenfalls durch Halogen und Alkyl mit 1 bis 4 Kohlenstoffatomen substituiertes Phenyl substituiertes Phenyl; gegebenenfalls durch Alkyl substituiertes Isoxazolyl; 1,2,4-Triazol-1-yl, Imidazol-1-yl und Pyrazol-1-yl, gegebenenfalls durch Cyano oder Thiocyano substituiertes Alkyl, Alkenyl und Alkinyl; gegebenenfalls durch Alkyl, mit 1 bis 4 Kohlenstoffatomen, Alkenyl mit 2 bis 4 Kohlenstoffatomen, Halogenalkyl mit 1 bis 4 Kohlenstoff- und bis zu 5 gleichen oder verschiedenen Halogenatomen, Halogenalkenyl mit 2 bis 4 Kohlenstoff- und bis zu 5 gleichen oder verschiedenen Halogenatomen, Halogen, Alkoxy mit 1 bis 4 Kohlenstoffatomen sowie eine zwei- bis fünfgliedrige Methylenbrücke substituiertes Cycloalkyl oder Cycloalkenyl; Dihalogenalkyl; sowie die Gruppierungen

$-CH_2Az$ $-CH_2R^9$ $-CH_2OR^8$ $-CH_2OR^9$ $-CH_2SR^8$ $-CH_2SR^9$

$-CH_2-SO_nR^8$ $-CH_2SO_n-R^9$ $-CH_2OSOR^8$ $-CH_2OSOR^9$ $-CH_2OSO_2R^8$

$-CH_2OSO_2R^9$ $-CH_2OCOR^8$ $-CH_2OCOR^9$ $-OR^8$ $-OR^9$ $-SR^8$

$-SR^9$ $-NR^{10}R^{11}$ $-CH_2-O-$ 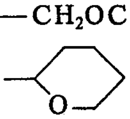

$R^8$ für gegebenenfalls durch Halogen, Cyano oder Thiocyano substituiertes Alkyl, Alkenyl, Alkinyl und Alkoxyalkyl steht,

$R^9$ für gegebenenfalls durch Halogen Alkyl, Alkoxy und Alkylthio mit jeweils 1 bis 4 Kohlenstoffatomen, Halogenalkyl, Halogenalkoxy und Halogenalkylthio mit jeweils 1 bis 4 Kohlenstoff- und 1 bis 3 gleichen oder verschiedenen Halogenatomen wie Fluor- und Chloratomen, Nitro, Cyano sowie gegebenenfalls durch Halogen und Alkyl mit 1 bis 4 Kohlenstoffatomen substituiertes Phenyl substituiertes Phenyl steht,

$R^{10}$ und $R^{11}$ gleich oder verschieden sind und für Wasserstoff, Alkyl, Alkenyl oder gegebenenfalls durch Halogen, Alkyl, Alkoxy und Alkylthio mit jeweils 1 bis 4 Kohlenstoffatomen, Halogenalkyl, Halogenalkoxy und Halogenalkylthio mit jeweils 1 bis 4 Kohlenstoff- und 1 bis 5 gleichen oder verschiedenen Halogenatomen, wie Fluor- und Chloratomen, Nitro, Cyano sowie gegebenenfalls durch Halogen und Alkyl mit 1 bis 4 Kohlenstoffatomen substituiertes Phenyl substituiertes Phenyl stehen,

Az für Pyrazol-1-yl, 1,2,4-Triazol-1-yl und Imidazol-1-yl steht,

n für 1 oder 2 steht,

sowie, wenn

Y für Az bzw. für $-CH_2-Az$ steht, deren physiologisch verträglichen Säureadditionssalze und Metallsalzkomplexe,

erhält, wenn man

a) Azine der Formel (II)

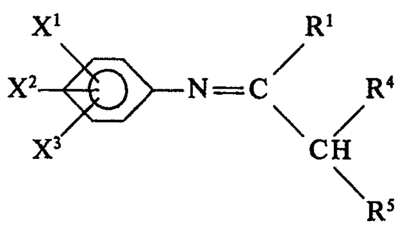

(II)

in welcher

$R^1$, $X^1$, $X^2$ und $X^3$ die oben angegebene Bedeutung haben, und

$R^4$ für Wasserstoff, Alkyl, Alkoxy, Alkoxyalkyl, Hydroxyalkyl oder Benzyl steht und

$R^5$ für Wasserstoff, Alkyl, Alkenyl, Alkoxy, Alkoxyalkyl, Alkylcarbonyl, Alkylcarbonylalkyl, Alkoxycarbonyl oder Alkoxycarbonylalkyl steht, oder

$R^1$ und $R^4$ gemeinsam mit der angrenzenden C—C-Bindung für einen gegebenenfalls ein- oder mehrfach durch Alkyl mit 1 bis 4 Kohlenstoffatomen, Halogen und Hydroxy substituierten gesättigten oder ungesättigten Ring stehen, der auch Heteroatome enthalten kann, oder

$R^4$ und $R^5$ gemeinsam mit dem angrenzenden Kohlenstoffatom für einen gegebenenfalls ein- oder mehrfach durch Alkyl mit 1 bis 4 Kohlenstoffatomen, Halogen und Hydroxy substituierten, gesättigten oder ungesättigten Ring stehen, der auch Heteroatome enthalten kann,

mit Säure-Derivaten der Formel (III)

$$Hal-CO-Y \qquad (III),$$

in welcher

Hal für Chlor oder Brom steht, und

Y die oben angegebene Bedeutung hat,

gegebenenfalls in Gegenwart eines Verdünnungsmittels umsetzt und die entstehenden Acetanilide der Formel (IV)

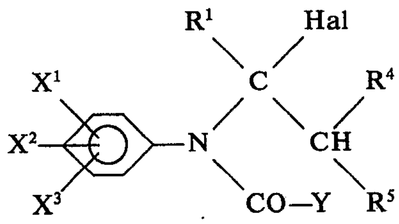

(IV)

in welcher

Hal, $R^1$, $R^4$, $R^5$, $X^1$, $X^2$, $X^3$ und Y die oben angegebene Bedeutung haben,

ohne vorherige Isolierung, gegebenenfalls in Gegenwart einer Base, der Halogenwasserstoffeliminierung unterwirft, oder

b) Carbamidsäurechloride der Formel (V)

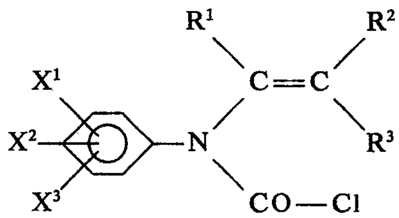

(V)

in welcher

$R^1$, $R^2$, $R^3$, $X^1$, $X^2$ und $X^3$ die oben angegebene Bedeutung haben,

mit Verbindungen der Formel (VI)

B—Y' (VI),

in welcher

Y' für Az sowie die Gruppierungen $-OR^8$, $-OR^9$, $-SR^8$, $-SR^9$ und $-NR^{10}R^{11}$ steht, wobei Az, $R^8$, $R^9$, $R^{10}$ und $R^{11}$ die oben angegebene Bedeutung haben und

B für Wasserstoff oder ein Alkalimetall steht,

gegebenenfalls in Gegenwart eines Verdünnungsmittels und gegebenenfalls in Gegenwart eines Säurebinders umsetzt, oder

c) Chloracetamide der Formel (VII)

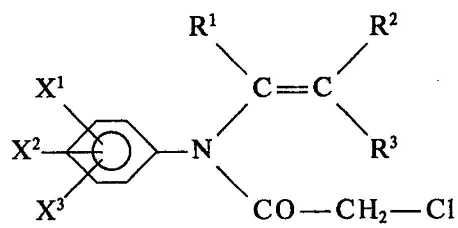

(VII)

in welcher

$R^1$, $R^2$, $R^3$, $X^1$, $X^2$ und $X^3$ die oben angegebene Bedeutung haben,

mit Verbindungen der Formel (VIII)

B—Y'' (VIII),

in welcher

Y'' für Az sowie die Gruppierungen $-OR^8$, $-OR^9$, $-SR^8$, $-SR^9$, $-OCOR^9$, $-OSOR^8$, $-OSOR^9$, $OSO_2R^8$, $-OSO_2R^9$ steht, wobei
Az, B, $R^8$ und $R^9$ die oben angegebene Bedeutung haben,

gegebenenfalls in Gegenwart eines Verdünnungsmittels und gegebenenfalls in Gegenwart eines Säurebindemittels umsetzt, oder

d) Hydroxyacetanilide der Formel (IX)

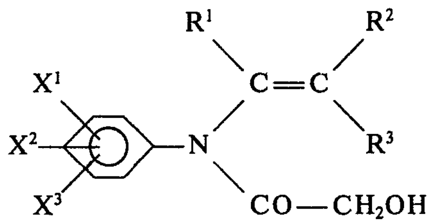

(IX)

in welcher

$R^1$, $R^2$, $R^3$, $X^1$, $X^2$ und $X^3$ die oben angegebene Bedeutung haben,

gegebenenfalls nach Aktivierung mittels Alkalimetall, mit Halogeniden der Formel (X)

$$Hal-Y''' \quad (X),$$

in welcher

Y''' für $R^8$ oder die Gruppierungen $-SO_2R^8$ und $-SO_2R^9$ steht, wobei
$R^8$ und $R^9$ die oben angegebene Bedeutung haben und
Hal für Chlor oder Brom steht,

in Gegenwart eines Verdünnungsmittels und gegebenenfalls in Gegenwart eines Säurebindemittels umsetzt, oder

e) erfindungsgemäße Acetanilide der Formel (XI)

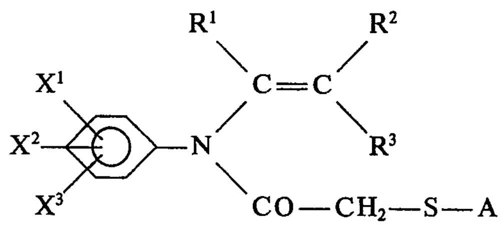

(XI)

in welcher

A für $R^8$ bzw. $R^9$ steht und
$R^1$, $R^2$, $R^3$, $R^8$, $R^9$, $X^1$, $X^2$ und $X^3$ die oben angegebene Bedeutung haben,

nach bekannten Methoden in üblicher Weise oxidiert.

Welche der angegebenen Verfahrensvarianten im Einzelfall angewendet wird, hängt insbesondere von der Zugänglichkeit der jeweils benötigten Ausgangsprodukte ab.

An die so erhaltenen Verbindungen der Formel (I) kann, wenn Y für Az bzw. $-CH_2-Az$ steht, eine Säure oder ein Metallsalz addiert werden.

Die neuen N-(1-Alkenyl)-carbonsäureanilide weisen starke fungizide Eigenschaften auf. Dabei zeigen überraschenderweise die erfindungsgemäßen Verbindungen eine höhere Wirkung als die aus dem Stand der Technik bekannten Verbindungen Zink-ethylen-1,2-bis-dithiocarbamidat, N-Chloracetyl-N-(2,6-dimethylphenyl)-alaninester, 2,6-Dimethyl-N-allyl-imidazolylacetanilid, 2,6-Dimethyl-N-allenylethoxyacetanilid und 2,6-Dimethyl-N-allenyl-N-(3-methyl-2-isoxazolylcarbonyl)-anilin. Die erfindungsgemäßen Stoffe stellen somit eine Bereicherung der Technik dar.

Die erfindungsgemäßen N-(1-Alkenyl)-carbonsäureanilide sind durch die Formel (I) allgemein definiert. In dieser Formel stehen vorzugsweise

$R^1$ für Wasserstoff oder geradkettiges bzw. verzweigtes Alkyl mit 1 bis 4 Kohlenstoffatomen;

$R^2$ für Wasserstoff, geradkettiges oder verzweigtes Alkyl mit 1 bis 4 Kohlenstoffatomen, Alkoxy mit 1 bis 4 Kohlenstoffatomen, Hydroxyalkyl mit 1 bis 4 Kohlenstoffatomen, Alkoxyalkyl mit 1 bis 4 Kohlenstoffatomen im Alkylteil und 1 bis 4 Kohlenstoffatomen im Alkoxyteil oder für Benzyl;

$R^3$ für Wasserstoff, geradkettiges oder verzweigtes Alkyl mit 1 bis 4 Kohlenstoffatomen, Alkoxy mit 1 bis 4 Kohlenstoffatomen, Alkoxyalkyl mit 1 bis 4 Kohlenstoffatomen im Alkylteil und 1 bis 4 Kohlenstoffatomen im Alkoxyteil, Alkylcarbonyl mit 1 bis 4 Kohlenstoffatomen im Alkylteil, Alkylcarbonylalkyl mit 1 bis 4 Kohlenstoffatomen in jedem Alkylteil, Alkoxycarbonyl mit 1 bis 4 Kohlenstoffatomen im Alkoxyteil, Alkoxycarbonylalkyl mit 1 bis 4 Kohlenstoffatomen im Alkoxyteil und 1 bis 4 Kohlenstoffatomen im Alkylteil oder für geradkettiges oder verzweigtes Alkenyl mit 2 bis 6 Kohlenstoffatomen;

oder

$R^1$ und $R^2$ gemeinsam mit der C=C-Doppelbindung für einen gegebenenfalls ein- oder mehrfach substituierten, einfach oder mehrfach ungesättigten, 5- oder 6gliedrigen Ring, der ein oder zwei Heteroatome enthalten kann, wobei als Substituenten beispielsweise genannt seien: Alkyl mit 1 bis 4 Kohlenstoffatomen, Halogen und Hydroxy;

oder

$R^2$ und $R^3$ gemeinsam mi dem angrenzenden Kohlenstoffatom für einen gegebenenfalls ein- oder mehrfach substituierten, gesättigten oder ungesättigten, 5- oder 6gliedrigen Ring, der ein oder zwei Heteroatome enthalten kann, wobei als Substituenten beispielsweise genannt seien: Alkyl mit 1 bis 4 Kohlenstoffatomen, Halogen und Hydroxy;

$X^1$ für Wasserstoff, geradkettiges oder verzweigtes Alkyl mit 1 bis 4 Kohlenstoffatomen, Fluor, Chlor oder Brom;

$X^2$ für Wasserstoff, geradkettiges oder verzweigtes Alkyl mit 1 bis 4 Kohlenstoffatomen, Fluor, Chlor oder Brom; und

$X^3$ für Wasserstoff und geradkettiges Alkyl mit 1 bis 4 Kohlenstoffatomen, Fluor, Chlor oder Brom,

Y für Furyl, Tetrahydrofuryl, Thienyl, Tetrahydrothienyl, für gegebenenfalls substituiertes Phenyl, wobei als Substituenten vorzugsweise genannt seien: Halogen, Alkyl, Alkoxy und Alkylthio mit jeweils 1 bis 4 Kohlenstoffatomen, Halogenalkyl, Halogenalkoxy und Halogenalkylthio mit jeweils 1 bis 4 Kohlenstoff- und 1 bis 5 gleichen oder verschiedenen Halogenatomen, wie Fluor- und Chloratomen, Nitro, Cyano sowie gegebenenfalls durch Halogen und Alkyl mit 1 bis 4 Kohlenstoffatomen substituiertes Phenyl, für gegebenenfalls durch Methyl oder Ethyl substituiertes Isoxazolyl, für 1,2,4-Triazol-1-yl, Imidazol-1-yl und Pyrazol-1-yl, für gegebenenfalls durch Cyano oder Thiocyano substituiertes Alkyl mit 1 bis 4 Kohlenstoffatomen sowie Alkenyl und Alkinyl mit jeweils 2 bis 4 Kohlenstoffatomen, für gegebenenfalls substituiertes Cycloalkyl mit 3 bis 7 Kohlenstoffatomen oder Cycloalkenyl mit 4 bis 7 Kohlenstoffatomen, wobei als Substituenten vorzugsweise infrage kommen: Alkyl mit 1 bis 4 Kohlenstoffatomen, Alkenyl mit 2 bis 4 Kohlenstoffatomen, Halogenalkyl mit 1 bis 4 Kohlenstoff- und bis zu 5 gleichen oder verschiedenen Halogenatomen, Halogenalkenyl mit 2 bis 4 Kohlenstoff- und bis zu 5 gleichen oder verschiedenen Halogenatomen, Halogen, Alkoxy mit 1 bis 4 Kohlenstoffatomen sowie eine zwei- bis fünfgliedrige Methylenbrükke; ferner für Dihalogenalkyl mit 1 bis 2 Kohlenstoffatomen, wobei als Halogenatome vorzugsweise Fluor, Chlor und Brom genannt seien; sowie für die Gruppierungen

$-CH_2Az$ $-CH_2R^9$ $-CH_2OR^8$ $-CH_2OR^9$ $-CH_2SR^8$ $-CH_2SR^9$

$-CH_2-SO_nR^8$ $-CH_2SO_n-R^9$ $-CH_2OSOR^8$ $-CH_2OSOR^9$ $-CH_2OSO_2R^8$

$-CH_2OSO_2R^9$ $-CH_2OCOR^8$ $-CH_2OCOR^9$ $-OR^8$ $-OR^9$ $-SR^8$

$-SR^9$ $-NR^{10}R^{11}$ und $-CH_2-O-$ 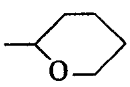

$R^8$ für gegebenenfalls durch Halogen, insbesondere Fluor, Chlor oder Brom, Cyano und Thiocyano substituiertes Alkyl mit 1 bis 4 und Alkenyl sowie Alkinyl mit jeweils 2 bis 4 Kohlenstoffatomen und für Alkoxyalkyl mit 1 bis 4 Kohlenstoffatomen in jedem Alkylteil;

$R^9$ für gegebenenfalls substituiertes Phenyl, wobei als Phenylsubstituenten vorzugsweise die bei Y bereits genannten infrage kommen;

$R^{10}$ und $R^{11}$ für Wasserstoff, Alkyl mit 1 bis 4 Kohlenstoffatomen, Alkenyl mit 2 bis 4 Kohlenstoffatomen sowie für gegebenenfalls substituiertes Phenyl, wobei als Phenylsubstituenten vorzugsweise die bei Y bereits genannten infrage kommen;

Az für Pyrazol-1-yl, 1,2,4-Triazol-1-yl und Imidazol-1-yl; und der Index

n für 1 oder 2.

Besonders bevorzugt sind diejenigen Verbindungen der Formel (I), in denen

$R^1$ für Wasserstoff, Methyl, Ethyl oder Isopropyl steht;
$R^2$ für Wasserstoff, Methyl, Ethyl, Isopropyl, Methoxy, Ethoxy, Isopropoxy, Methoxymethyl, Ethoxymethyl, Hydroxyisopropyl oder Benzyl steht;
$R^3$ für Wasserstoff, Methyl, Ethyl, Isopropyl, Methoxy, Ethoxy, Isopropoxy, Methoxymethyl, Ethoxymethyl, Methylcarbonyl, Ethylcarbonyl, Methylcarbonylmethyl, Methoxycarbonyl, Ethoxycarbonyl, Methoxycarbonylmethyl sowie für geradkettiges oder verzweigtes Alkenyl mit 2 bis 4 Kohlenstoffatomen steht;
oder
$R^1$ und $R^2$ gemeinsam mit der C=C-Doppelbindung für einen gegebenenfalls durch Methyl, Ethyl, Hydroxy und/oder Chlor substituierten, einfach oder mehrfach ungesättigten 5- oder 6gliedrigen Ring stehen, der ein oder zwei Heteroatome, wie insbesondere Sauerstoff, Stickstoff oder Schwefel, enthalten kann;
oder
$R^2$ und $R^3$ gemeinsam mit dem angrenzenden Kohlenstoffatom für einen gegebenenfalls durch Methyl, Ethyl, Hydroxy und/oder Chlor substituierten, gesättigten oder ungesättigten 5- oder 6gliedrigen Ring stehen, der ein oder zwei Heteroatome, wie insbesondere Sauerstoff, Stickstoff oder Schwefel, enthalten kann;
$X^1$ für Wasserstoff, Methyl, Ethyl, Isopropyl, Isobutyl, sek.-Butyl, tert.-Butyl, Chlor oder Fluor steht;
$X^2$ für Wasserstoff, Methyl, Ethyl, Isopropyl, Isobutyl, sek.-Butyl, tert.-Butyl, Chlor oder Fluor steht;
$X^3$ für Wasserstoff, Methyl, Ethyl, Isopropyl, Isobutyl, sek.-Butyl, tert.-Butyl, Chlor oder Fluor steht;
Y für 2-Furyl, 2-Thienyl, 2-Tetrahydrofuryl; für gegebenenfalls durch Fluor, Chlor, Methyl, Methoxy, Methylthio, Trifluormethyl, Trifluormethoxy, Trifluormethylthio, Nitro, Cyano, Phenyl und/oder Chlorphenyl substituiertes Phenyl; für 5-Methylisoxazol-3-yl, Pyrazol-1-yl, 1,2,4-Triazol-1-yl, Imidazol-1-yl; für gegebenenfalls durch Cyano substituiertes Methyl, Ethyl, Allyl oder Propargyl; für Cyclopropyl, Cyclobutyl, Cyclopentyl, Cyclohexyl, Cyclobutenyl, Cyclopentenyl, Cyclohexenyl oder Cycloheptenyl steht, die jeweils durch Methyl, Ethyl, Vinyl, Allyl, Fluor, Chlor, Chlormethyl, Trifluormethyl, Dichlorvinyl, Methoxy und Trimethylen substituiert sein können; für Dichlormethyl sowie für die Gruppierungen
$-CH_2Az$, $-CH_2OR^8$, $-CH_2OR^9$, $-CH_2SR^8$, $-CH_2SR^9$, $-CH_2SO_nR^8$, $-CH_2SO_nR^9$, $-CH_2OSO_2R^8$, $-CH_2OSO_2R^9$, $-CH_2R^9$, $-CH_2OCOR^8$, $-CH_2OCOR^9$, $-OR^9$, $-SR^8$, $-SR^9$ und $-NR^{10}R^{11}$ steht;
$R^8$ für Methyl, Ethyl, Allyl, Propyl, Propargyl, Methoxymethyl, Ethoxymethyl oder Ethoxyethyl steht;
$R^9$ für gegebenenfalls substituiertes Phenyl steht, wobei als Substituenten insbeondere die bei Y bereits genannten infrage kommen;
$R^{10}$ und $R^{11}$ gleich oder verschieden sind und für Wasserstoff, Methyl, Ethyl, Isopropyl, Vinyl, Allyl, oder gegebenenfalls durch Fluor, Chlor, Methyl, Methoxy, Methylthio und/oder Trifluormethyl substituiertes Phenyl stehen;
Az und n die oben angegebene Bedeutung haben.

Im einzelnen seien außer den bei den Herstellungsbeispielen genannten Verbindungen die folgenden Verbindungen der allgemeinen Formel (I) genannt:

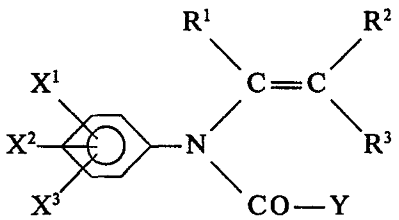

(I)

| $X^1$ | $X^2$ | $X^3$ | $-CR^1{=}CR^2R^3$ | Y |
|---|---|---|---|---|
| H | 2-$CH_3$ | 6-$CH_3$ | $-C(CH_3){=}CH_2$ | $-CH_2-$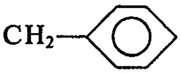 |
| H | 2-$CH_3$ | 6-$CH_3$ | $-C(CH_3){=}CH_2$ | $-CH_2-SCH_3$ |
| H | 2-$CH_3$ | 6-$CH_3$ | $-C(CH_3){=}CH_2$ | $-CH_2-SO-CH_3$ |
| H | 2-$CH_3$ | 6-$CH_3$ | $-C(CH_3){=}CH_2$ | $-CH_2-SO_2-CH_3$ |
| H | 2-$CH_3$ | 6-$CH_3$ | $-C(CH_3){=}CH_2$ | $-CH_2-SCN$ |

Fortsetzung

| $X^1$ | $X^2$ | $X^3$ | $-CR^1{=}CR^2R^3$ | Y |
|---|---|---|---|---|
| H | 2-$CH_3$ | 6-$CH_3$ | $-C(CH_3){=}CH_2$ | 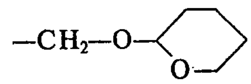 |
| H | 2-$CH_3$ | 6-$CH_3$ | 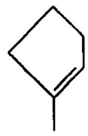 | 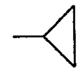 |
| H | 2-$CH_3$ | 6-$CH_3$ | $-C(CH_3){=}CH_2$ | 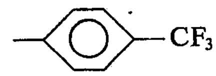 |
| H | 2-Cl | 6-$CH_3$ | 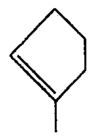 | 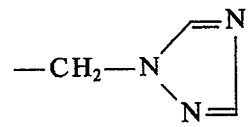 |
| H | 2-$CH_3$ | 6-$CH_3$ | 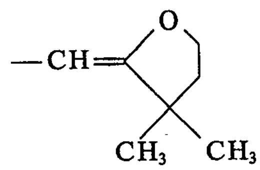 | $-CH_2OCH_3$ |
| H | 2-$CH_3$ | 6-$CH_3$ | 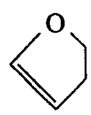 | 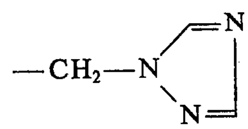 |
| 4-$CH_3$ | 2-$CH_3$ | 6-$CH_3$ | 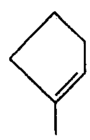 | $-CH_2OCH_3$ |
| H | 2-$CH_3$ | 6-$CH_3$ | 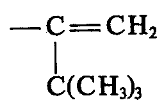 | $-CH_2OCH_3$ |
| H | 2-$CH_3$ | 6-$CH_3$ | 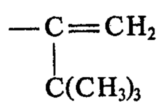 | 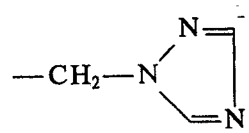 |
| H | 2-$CH_3$ | 6-$CH_3$ | 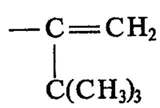 | $-CH_2OCH_3$ |
| H | 2-$CH_3$ | 6-$CH_3$ | $-CH{=}CH-C_2H_5$ | $-CH_2OCH_3$ |
| H | 2-$CH_3$ | 6-$CH_3$ | 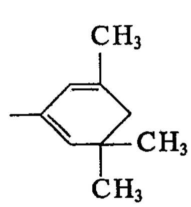 | $-CH_2OCH_3$ |
| H | 2-$CH_3$ | 6-$CH_3$ | 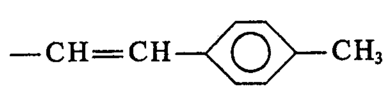 | 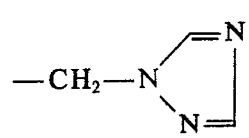 |

Verwendet man beispielsweise N-Isopropyliden-2,6-dimethylanilin und Methoxyessigsäurechlorid als Ausgangsstoffe, so kann der Reaktionsablauf durch das folgende Formelschema wiedergegeben werden (Verfahren a):

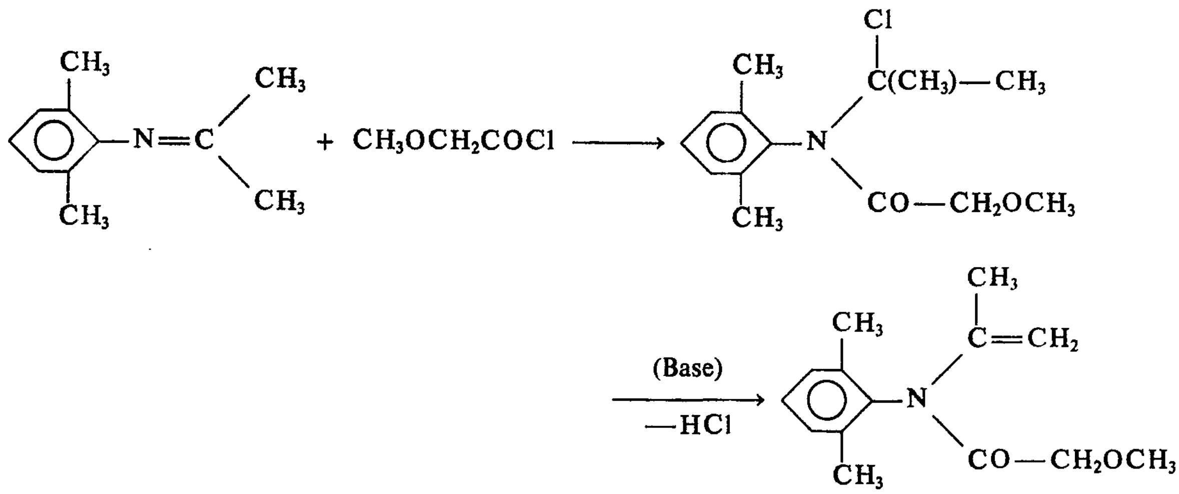


Verwendet man beispielsweise N-Chlorcarbonyl-N-(1-methylvinyl)-2,6-dimethylanilin und 1,2,4-Triazol als Ausgangsstoffe, so kann der Reaktionsablauf durch das folgende Formelschema wiedergegeben werden (Verfahren b):

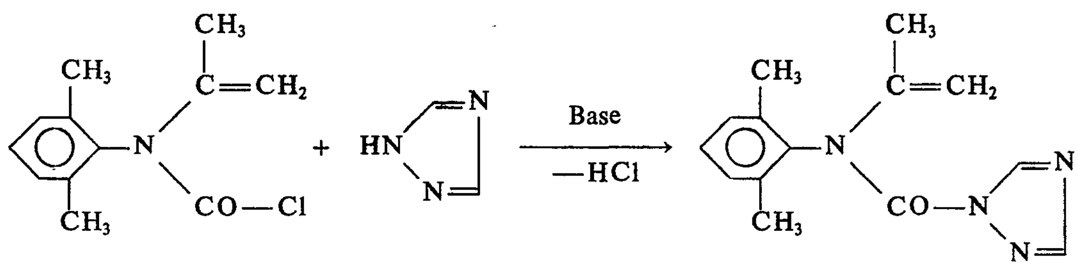


Verwendet man beispielsweise N-Chloracetyl-N-(1-methylvinyl)-2,6-dimethylanilin und Pyrazol als Ausgangsstoffe, so kann der Reaktionsablauf durch das folgende Formelschema wiedergegeben werden (Verfahren c):

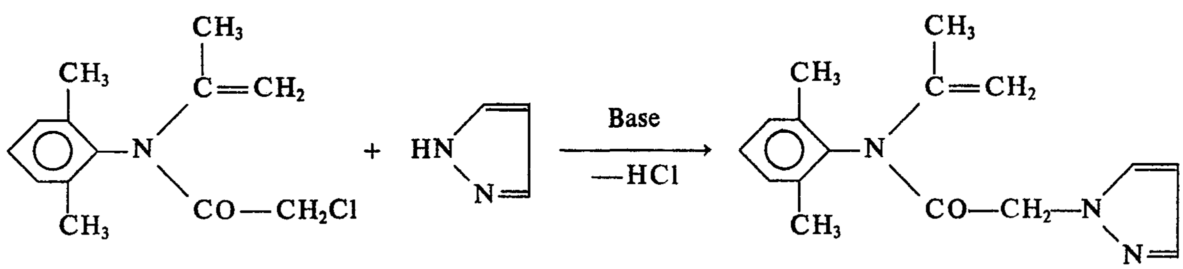


Verwendet man beispielsweise N-Hydroxyacetyl-N-vinyl-2,6-dimethylanilin und Methansulfonsäurechlorid als Ausgangsstoffe, so kann der Reaktionsablauf durch das folgende Formelschema wiedergegeben werden (Verfahren d):

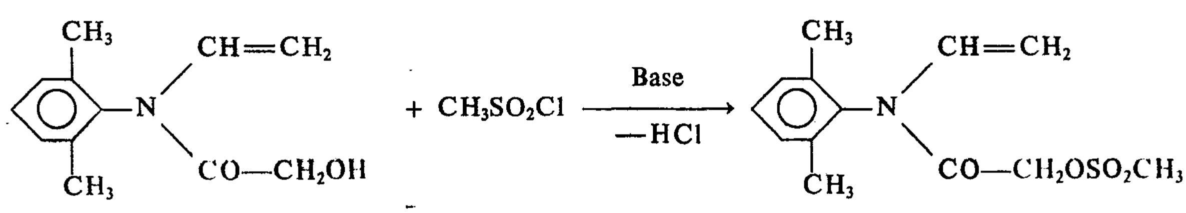

Verwendet man beispielsweise 1 Mol N-Methylthioacetyl-N-vinyl-2,6-dimethylanilin und 2 Mol m-Chlorperbenzoesäure als Ausgangsstoffe, so kann der Reaktionsablauf durch das folgende Formelschema wiedergegeben werden (Verfahren e):

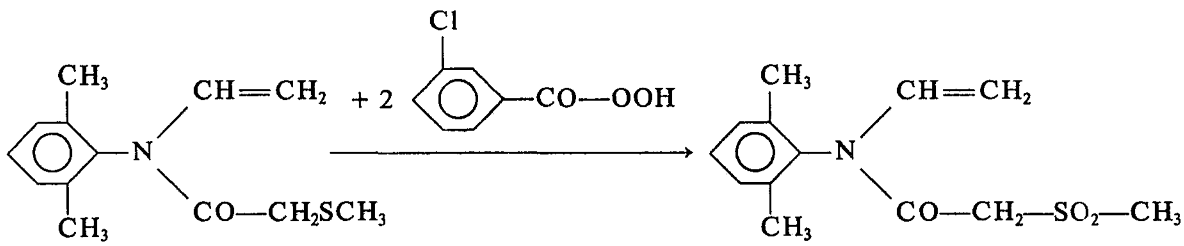

Die für das erfindungsgemäße Verfahren (a) als Ausgangsstoffe zu verwendenden Azine sind durch die Formel (II) allgemein definiert. In dieser Formel stehen $R^1$, $X^1$, $X^2$ und $X^3$ vorzugsweise für die Reste, die bereits im Zusammenhang mit der Beschreibung der erfindungsgemäßen Stoffe der Formel (I) vorzugsweise für diese Substituenten genannt wurden.

$R^4$ steht in der Formel (II) vorzugsweise für Wasserstoff, geradkettiges oder verzweigtes Alkyl mit 1 bis 4 Kohlenstoffatomen, Alkoxy mit 1 bis 4 Kohlenstoffatomen, Hydroxyalkyl mit 1 bis 4 Kohlenstoffatomen, Alkoxyalkyl mit 1 bis 4 Kohlenstoffatomen im Alkylteil und 1 bis 4 Kohlenstoffatomen im Alkoxyteil oder für Benzyl.

$R^5$ steht vorzugsweise für Wasserstoff, geradkettiges oder verzweigtes Alkyl mit 1 bis 4 Kohlenstoffatomen, Alkoxy mit 1 bis 4 Kohlenstoffatomen, Alkoxyalkyl mit 1 bis 4 Kohlenstoffatomen im Alkylteil und 1 bis 4 Kohlenstoffatomen im Alkoxyteil, Alkylcarbonyl mit 1 bis 4 Kohlenstoffatomen im Alkylteil, Alkylcarbonylalkyl mit 1 bis 4 Kohlenstoffatomen in jedem Alkylteil, Alkoxycarbonyl mit 1 bis 4 Kohlenstoffatomen im Alkoxyteil, Alkoxycarbonylalkyl mit 1 bis 4 Kohlenstoffatomen im Alkoxyteil und 1 bis 4 Kohlenstoffatomen im Alkylteil oder für geradkettiges oder verzweigtes Alkenyl mit 2 bis 6 Kohlenstoffatomen.

Außerdem stehen $R^1$ und $R^4$ in der Formel (II) auch gemeinsam mit der angrenzenden C—C-Bindung vorzugsweise für einen gegebenenfalls ein- oder mehrfach substituierten, gesättigten oder ungesättigten 5- oder 6gliedrigen Ring, der ein oder zwei Heteroatome enthalten kann, wobei als Substituenten beispielsweise genannt seien: Alkyl mit 1 bis 4 Kohlenstoffatomen, Halogen und Hydroxy.

Ferner stehen in der Formel (II) auch $R^4$ und $R^5$ gemeinsam mit dem angrenzenden Kohlenstoffatom vorzugsweise für einen gegebenenfalls ein- oder mehrfach substituierten, gesättigten oder ungesättigten, 5- oder 6gliedrigen Ring der ein oder zwei Heteroatomen enthalten kann, wobei als Substituenten beispielsweise genannt seien: Alkyl mit 1 bis 4 Kohlenstoffatomen, Halogen und Hydroxy.

Die Azine der Formel (II) sind bekannt oder lassen sich in einfacher Weise nach im Prinzip bekannten Verfahren herstellen (vgl. DE-OS 1 670 859 und DE-OS 3 011 084 sowie DE-OS 3 120 990).

Die außerdem für das erfindungsgemäße Verfahren (a) als Ausgangsstoffe zu verwendenden Säure-Derivate sind durch die Formel (III) allgemein definiert. In dieser Formel steht Y vorzugsweise für diejenigen Reste, die bereits im Zusammenhang mit der Beschreibung der erfindungsgemäßen Stoffe der Formel (I) vorzugsweise für diesen Substituenten genannt wurden.

Die Säure-Derivate der Formel (III) sind bekannte Verbindungen der organischen Chemie, bzw. können sie in üblicher Weise erhalten werden.

Die bei der Durchführung des erfindungsgemäßen Verfahrens (b) als Ausgangsstoffe zu verwendenden Carbamidsäurechloride sind durch die Formel (V) allgemein definiert. In dieser Formel stehen $R^1$, $R^2$, $R^3$, $X^1$, $X^2$ und $X^3$ vorzugsweise für diejenigen Reste, die bereits im Zusammenhang mit der Beschreibung der erfindungsgemäßen Stoffe der Formel (I) vorzugsweise für diese Substituenten genannt wurden.

Die Carbamidsäurechloride der Formel (V) sind noch nicht bekannt. Sie werden erhalten, indem man Azide der Formel (II) mit Phosgen gemäß Verfahren (a) umsetzt.

Die außerdem für das erfindungsgemäße Verfahren (b) als Ausgangsstoffe zu verwendenden Verbindungen sind durch die Formel (VI) allgemein definiert. In dieser Formel stehen Az, $R^8$, $R^9$, $R^{10}$ und $R^{11}$ vorzugsweise für diejenigen Reste, die bereits im Zusammenhang mit der Beschreibung der erfindungsgemäßen Stoffe der Formel (I) vorzugsweise für diese Substituenten genannt wurden und B steht vorzugsweise für Wasserstoff, Natrium und Kalium.

Die Verbindungen der Formel (VI) sind allgemein bekannte Verbindungen der organischen Chemie.

Die bei der Durchführung des erfindungsgemäßen Verfahrens (c) als Ausgangsstoffe zu verwendenden Chloracetamide sind durch die Formel (VII) allgemein definiert. In dieser Formel stehen $R^1$, $R^2$,

$R^3$, $X^1$, $X^2$ und $X^3$ vorzugsweise für diejenigen Reste, die bereits im Zusammenhang mit der Beschreibung der erfindungsgemäßen Stoffe der Formel (I) vorzugsweise für diese Substituenten genannt wurden.

Die Chloracetamide der Formel (VII) sind noch nicht bekannt, sie sind jedoch Gegenstand einer eigenen Anmeldung, die noch nicht offengelegt ist (vgl. DE-OS 3 120 990). Sie können nach dem dort angegebenen Verfahren erhalten werden, indem man Azine der Formel (II) entsprechend den Angaben unter Verfahren (a) mit Chloracethalogeniden umsetzt.

Die außerdem für das erfindungsgemäße Verfahren (c) als Ausgangsstoffe zu verwendenden Verbindungen sind durch die Formel (VIII) allgemein definiert. In dieser Formel stehen Az, $R^8$ und $R^9$ vorzugsweise für diejenigen Reste, die bereits im Zusammenhang mit der Beschreibung der erfindungsgemäßen Stoffe der Formel (I) vorzugsweise für diese Substituenten genannt wurden und B vorzugsweise für Wasserstoff, Natrium und Kalium.

Die Verbindungen der Formel (VIII) sind allgemein bekannte Verbindungen der organischen Chemie.

Die bei der Durchführung des erfindungsgemäßen Verfahrens (d) als Ausgangsstoffe zu verwendenden Hydroxyacetanilide sind durch die Formel (IX) allgemein definiert. In dieser Formel stehen $R^1$, $R^2$, $R^3$, $X^1$, $X^2$ und $X^3$ vorzugsweise für diejenigen Reste, die bereits im Zusammenhang mit der Beschreibung der erfindungsgemäßen Stoffe der Formel (I) vorzugsweise für diese Substituenten genannt wurden.

Die Hydroxyacetanilide der Formel (IX) sind noch nicht bekannt, sie können jedoch in allgemein bekannter Art und Weise erhalten werden, indem man erfindungsgemäße Acyloxyacetyl-anilide der Formel (XII)

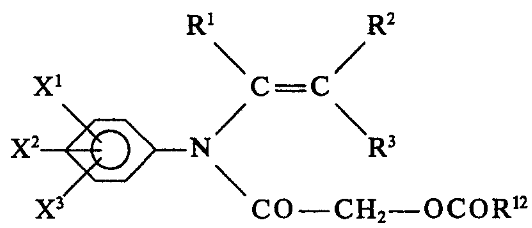

(XII)

in welcher

$R^1$, $R^2$, $R^3$, $X^1$, $X^2$ und $X^3$ die bei Formel I angegebene Bedeutung haben und
$R^{12}$ für Alkyl mit 1 bis 4 Kohlenstoffatomen steht,

mit Natron- oder Kalilauge bzw. mit einem Alkalialkoholat eines niederen Alkohols, wie z. B. Natriummethylat oder Natriumethylat, bei Temperaturen zwischen 20 und 40°C verseift und nach Ansäuern in üblicher Weise die Verbindungen der Formel (IX) isoliert.

Die außerdem für das erfindungsgemäße Verfahren (d) als Ausgangsstoffe zu verwendenden Halogenide sind durch die Formel (X) allgemein definiert. In dieser Formel stehen $R^8$ und $R^9$ vorzugsweise für diejenigen Reste, die bereits im Zusammenhang mit der Beschreibung der erfindungsgemäßen Stoffe der Formel (I) vorzugsweise für diesen Substituenten genannt wurden.

Die Halogenide der Formel (X) sind allgemein bekannte Verbindungen der organischen Chemie.

Die bei der Durchführung des erfindungsgemäßen Verfahrens (e) als Ausgangsstoffe zu verwendenden Acetanilide sind durch die Formel (XI) allgemein definiert. Die Verbindungen der Formel (XI) sind erfindungsgemäße Stoffe.

Die erfindungsgemäße Oxidation gemäß Verfahren (e) erfolgt durch die Umsetzung mit üblichen anorganischen oder organischen Oxidationsmitteln. Hierzu gehören vorzugsweise organische Persäuren, wie z. B. Peressigsäure, p-Nitroperbenzoesäure, m-Chlorperbenzoesäure; anorganische Persäuren, wie z. B. Perjodsäure; weiterhin Wasserstoffperoxid in Eisessig oder Methanol.

Als Verdünnungsmittel kommen für das erfindungsgemäße Verfahren (a) inerte organische Lösungsmittel infrage. Hierzu gehören vorzugsweise Ketone wie Aceton und Methylethylketon; Nitrile wie Acetonitril; Ether wie Tetrahydrofuran oder Dioxan; Ester, wie Essigester; halogenierte Kohlenwasserstoffe, wie Methylenchlorid; sowie insbesondere aromatische Kohlenwasserstoffe wie Toluol und Benzol.

Die Reaktionstemperaturen können bei der Durchführung des erfindungsgemäßen Verfahrens (a) in einem größeren Bereich variiert werden. Im allgemeinen arbeitet man bei Temperaturen von −20 bis +150°C, vorzugsweise von −20 bis +100°C.

Die Halogenwasserstoffeliminierung gemäß dem erfindungsgemäßen Verfahren (a) erfolgt in allgemein üblicher und bekannter Weise durch Erhitzen des Reaktionsgemisches unter Rückfluß oder durch Zugabe einer Base bei den oben genannten Temperaturen.

Als Base können alle üblicherweise verwendbaren anorganischen oder organischen Säureakzeptoren eingesetzt werden. Hierzu gehören vorzugsweise Alkalicarbonate, wie Natrium- und Kaliumcarbonat oder Natriumhydrogencarbonat; ferner niedere tertiäre Alkylamine, Aralkylamine, aromatische Amine oder Cycloalkylamine, wie z. B. Triethylamin, Dimethylbenzylamin, Pyridin, 1,5-Diazabicyclo-[4.3.0]-non-5-en (DBN) und 1,8-Diazabicyclo-[5.4.0]-undec-7-en (DBU). Es ist aber auch möglich, einen entsprechenden Überschuß an Azin der Formel (II) einzusetzen.

Bei der Durchführung des erfindungsgemäßen Verfahrens (a) arbeitet man vorzugsweise in äquimolaren Mengen. Die Isolierung der Endprodukte erfolgt in allgemein üblicher Art und Weise.

Als Verdünnungsmittel kommen für die erfindungsgemäßen Verfahren (b) und (c) vorzugsweise inerte organische Lösungsmittel infrage. Hierzu gehören vorzugsweise Ketone wie Diethylketon, insbesondere Aceton und Methylethylketon; Nitrile wie Propionitril, insbesondere Acetonitril; Ether wie Tetrahydrofuran oder Dioxan; aliphatische und aromatische Kohlenwasserstoffe wie Petrolether, Benzol, Toluol oder Xylol; halogenierte Kohlenwasserstoffe wie Methylenchlorid, Tetrachlorkohlenstoff, Chloroform oder Chlorbenzol; Ester wie Essigester und Formamide wie insbesondere Dimethylformamid.

Die erfindungsgemäßen Verfahren (b) und (c) können gegebenenfalls in Gegenwart von Säurebindern durchgeführt werden. Als solche können die üblichen Säurebindemittel verwendet werden. Hierzu gehören vorzugsweise die beim Verfahren (a) bereits genannten Stoffe. Gegebenenfalls kann auch ein Überschuß an Azol verwendet werden.

Die Reaktionstemperaturen können bei den Verfahren (b) und (c) in einem größeren Bereich variiert werden. Im allgemeinen arbeitet man von etwa 20 bis 150° C, vorzugsweise von 60 bis 120° C. Bei Anwesenheit eines Verdünnungsmittels wird zweckmäßigerweise beim Siedepunkt des jeweiligen Verdünnungsmittels gearbeitet.

Bei der Durchführung der erfindungsgemäßen Verfahren (b) und (c) setzt man auf 1 Mol der Verbindungen der Formeln (V) bzw. (VII) vorzugsweise etwa 1 bis 2 Mol der Verbindungen der Formeln (VI) bzw. (VIII) und gegebenenfalls etwa 1 bis 2 Mol Säurebinder ein. Die Isolierung der Verbindungen der Formel (I) erfolgt in üblicher Weise.

Als Verdünnungsmittel für das erfindungsgemäße Verfahren (d) kommen vorzugsweise inerte organische Lösungsmittel infrage. Hierzu gehören vorzugsweise die beim Verfahren (b) bereits genannten Lösungsmittel.

Die Umsetzung nach Verfahren (d) kann gegebenenfalls in Gegenwart eines Säurebinders durchgeführt werden. Man kann alle üblicherweise verwendbaren anorganischen oder oganischen Säurebinder zugeben. Hierzu gehören vorzugsweise die beim Verfahren (a) bereits genannten Verbindungen.

Die Reaktionstemperaturen können beim Verfahren (d) in einem größeren Bereich variiert werden. Im allgemeinen arbeitet man von 20 bis 150° C, vorzugsweise bei der Siedetemperatur des jeweiligen Verdünnungsmittels, beispielsweise von 60 bis 100° C.

Bei der Durchführung des erfindungsgemäßen Verfahrens (d) setzt man auf 1 Mol der Verbindungen der Formel (IX), gegebenenfalls nach Zugabe von 1 bis 2 Mol einer starken Base, wie z. B. eines Alkalihydrids, 1 Mol Halogenid der Formel (X) und gegebenenfalls 1 bis 2 Mol Säurebinder ein. Zur Isolierung der Endprodukte wird das Reaktionsgemisch vom Lösungsmittel befreit und der Rückstand mit Wasser und einem organischen Lösungsmittel versetzt. Die organische Phase wird abgetrennt und in üblicher Weise aufgearbeitet.

Nach einer bevorzugten Ausführungsform des Verfahrens (d) wird zweckmäßigerweise so verfahren, daß man von einem Hydroxyacetanilid der Formel (IX) ausgeht, letzteres in einem geeigneten Lösungsmittel mittels Alkalimetallhydrid oder -bromid in das Alkalimetall-alkanolat überführt, und letzteres ohne Isolierung sofort mit einem Halogenid der Formel (X) umsetzt, wobei unter Abspalten von Alkalihalogenid die erfindungsgemäßen Verbindungen der Formel (I) in einem Arbeitsgang erhalten werden.

Als Verdünnungsmittel kommen für das erfindungsgemäße Verfahren (e) vorzugsweise inerte organische Lösungsmittel infrage. Hierzu gehören vorzugsweise Ketone wie Diethylketon, insbesondere Aceton und Methylisobutylketon; Nitrile wie Propionitril, insbesondere Acetonitril; Ether wie Tetrahydrofuran oder Dioxan; aliphatische und aromatische Kohlenwasserstoffe wie Petrolether, Benzol, Toluol oder Xylol; halogenierte Kohlenwasserstoffe wie Methylenchlorid, Tetrachlorkohlenstoff, Chloroform oder Chlorbenzol; Ester wie Essigester; Alkohole, wie Methanol; und Carbonsäuren wie Essigsäure.

Die Reaktionstemperaturen können bei der Durchführung der Oxidation gemäß Verfahren (e) in einem größeren Bereich variiert werden. Im allgemeinen arbeitet man von etwa 20 bis 40° C, vorzugsweise von $-10$ bis $+30$° C.

Bei der Durchführung der erfindungsgemäßen Oxidation gemäß Verfahren (e) setzt man auf 1 Mol der erfindungsgemäßen Verbindungen der Formel (XI) 1 oder 2 Mol Oxidationsmittel ein. Bei der Anwendung von 1 Mol Oxidationsmittel wie m-Chlorperbenzoesäure in Methylenchlorid, bei Temperaturen von $-20$ bis $+20$° C, entstehen vorzugsweise die erfindungsgemäßen Verbindungen der Formel (I) mit $n=1$. Mit 2 Mol Oxidationsmittel und höheren Temperaturen (0 bis 40° C) entstehen vorzugsweise die erfindungsgemäßen Verbindungen der Formel (I) mit $n=2$. Die Isolierung der Oxidationsprodukte erfolgt in üblicher Weise.

Die erfindungsgemäß herstellbaren Verbindungen der Formel (I) können für $Y=Az$ und $-CH_2-Az$ in Säureadditionssalze bzw. Metallsalz-Komplexe überführt werden.

Zur Herstellung von physiologisch verträglichen Säureadditionssalzen der Verbindungen der Formel (I) können vorzugsweise folgende Säuren in Frage kommen:

Halogenwasserstoffsäuren wie z. B. Chlorwasserstoffsäure und Bromwasserstoffsäure, insbesondere Chlorwasserstoffsäure,
weiterhin Phosphorsäure, Salpetersäure, Schwefelsäure,
mono- und bifunktionelle Carbonsäuren und Hydroxycarbonsäuren wie z. B. Essigsäure, Maleinsäure, Bernsteinsäure,
Sorbinsäure, Milchsäure, Sulfonsäuren wie z. B. p-Toluolsulfonsäure und 1,5-Naphthalindisulfonsäure.

Die Säureadditionsalze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, erhalten werden und in bekannter Weise, z. B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. bis II. sowie IV. bis VIII. Nebengruppe des Periodensystems nach Mendelejew in Frage, wobei Kupfer, Zink, Mangan, Magnesium, Zinn, Eisen und Nickel beispielhaft genannt seien.

Als Anionen der Salze können solche in Betracht kommen, die sich vorzugsweise von folgenden Säuren ableiten: Halogenwasserstoffsäuren wie z. B. Chlorwasserstoffsäure und Bromwasserstoffsäure, ferner Phosphorsäure, Salpetersäure und Schwefelsäure.

Die Metallsalz-Komplexe von Verbindungen der Formel (I) können in einfacher Weise nach üblichen Verfahren erhalten werden, so z. B. durch Lösen des Metallsalzes in Alkohol, z. B. Ethanol und Hinzufügen zu Verbindungen der Formel (I). Man kann Metallsalz-Komplexe in bekannter Weise, z. B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Oomyceten, z. B. gegen den Erreger der Kraut- und Braunfäule der Tomate und der Kartoffel (Phytophthora infestans) eingesetzt werden. Besonders hervorzuheben ist, daß die erfindungsgemäßen Wirkstoffe nicht nur eine protektive, sondern auch eine systemische Wirkung entfalten. So gelingt es, Pflanzen gegen Pilzbefall zu schützen, wenn man den Wirkstoff über den Boden und die Wurzel oder über das Saatgut den oberirdischen Teilen der Pflanze zuführt.

In entsprechenden Aufwandmengen zeigen die erfindungsgemäßen Verbindungen auch herbizide und pflanzenwachstumsregulierende Eigenschaften sowie Wirkung gegen Hygiene- und Vorratsschädlinge.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä. sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z. B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgase, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z. B. nichtionogene und anionische Emulgatoren, wie Polyoxyethylen-Fettsäureester, Polyoxyethylen-Fettalkohol-ether, z. B. Alkylarylpolyglykol-ether, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z. B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

## Herstellungsbeispiele

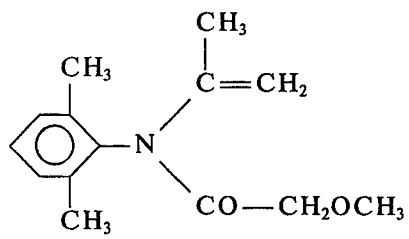

(Verfahren a)

Zu einer Lösung von 115 g (1 Mol) Methoxyessigsäurechlorid in 100 ml Toluol werden unter Rühren bei 0°C 166 g (1 Mol) N-Isopropyliden-2,6-dimethylanilin, gelöst in 300 ml Toluol, eingetropft. Man läßt 2 Stunden bei Raumtemperatur nachrühren und tropft dann bei 0°C 152 g (1 Mol) 2,3,4,6,7,8,9,10-Octahydro-[1,2,0]-azepin (DBU) zu. Man läßt 3 Stunden bei 20°C nachrühren, gießt das Gemisch in 2000 ml verdünnte, wäßrige Bicarbonatlösung, gibt 500 ml Essigester zu und rührt aus. Die organische Phase wird abgetrennt, mehrfach mit Wasser gewaschen und dann im Vakuum eingedampft. Der Rückstand wird im Hochvakuum fraktioniert destilliert. Man erhält 136 g (58,4% der Theorie) N-Methoxyacetyl-N-(1-methylvinyl)-2,6-dimethylanilin in Form eines farblosen Öls vom Siedepunkt 87—88°C/0,4 mbar.

Herstellung des Ausgangsproduktes

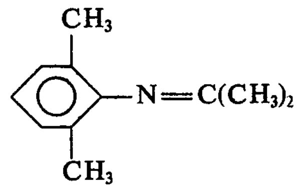

Ein Gemisch aus 121 g Dimethylanilin und 156 g (1,5 Mol) 2,2-Dimethoxypropan werden mit 0,1 g p-Toluolsulfonsäure versetzt und 30 Minuten unter Rückfluß erhitzt. Anschließend wird das entstandene Methanol abdestilliert.

Zur Vervollständigung der Reaktion werden nochmals 52 g (0,5 Mol) 2,2-Dimethoxypropan und 0,05 g p-Toluolsulfonsäure zugesetzt und das Reaktionsgemisch destilliert bis der Siedepunkt des 2,2-Dimethoxypropans (83°C) erreicht ist. Die verbleibende Lösung wird im Vakuum eingedampft. Man erhält 157 g (97% der Theorie) N-Isopropyliden-2,6-dimethylanilin in Form eines farblosen Öls.

Beispiel 2

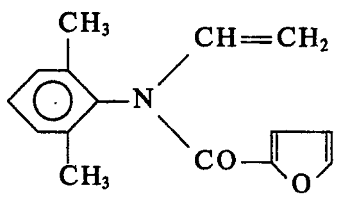

(Verfahren a)

Zu einer Lösung von 130,5 g (1 Mol) 2-Furylcarbonsäurechlorid in 100 ml Toluol werden unter Rühren bei 0°C 147,2 g (1 Mol) N-Ethyliden-2,6-dimethylanilin eingetropft. Man rührt 3 Stunden bei 20°C nach, tropft anschließend bei 20°C 101 g (1 Mol) Triethylamin zu und läßt 2 Stunden nachrühren. Man saugt vom ausgefallenen Triethylamin-hydrochlorid ab und wäscht die organische Phase mehrfach mit Wasser. Der nach dem Eindampfen der organischen Phase verbleibende Rückstand wird aus Tetrachlorkohlenstoff fraktioniert kristallisiert. Man erhält 49 g (20,4% der Theorie) N-(2-Furoyl)-N-vinyl-2,6-dimethylanilin in Form farbloser Kristalle vom Schmelzpunkt 90°C.

Herstellung des Ausgangsproduktes

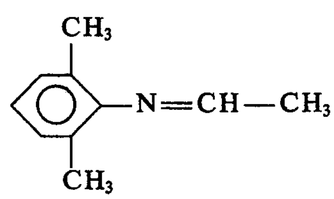

121 g (1 Mol) 2,6-Dimethylanilin in 200 ml Methylenchlorid werden mit 480 g (3 Mol) Natriumsulfat und 3 ml Dimethylbenzylamin versetzt. Dazu werden bei 0°C 88 g (2 Mol) Acetaldehyd zugegeben. Man läßt 4 Stunden bei 0°C rühren, filtriert und engt das Filtrat im Vakuum ein. Man erhält 140 g (95,2% der Theorie) N-Ethyliden-2,6-dimethylanilin als rötliches Öl.

Beispiel 3

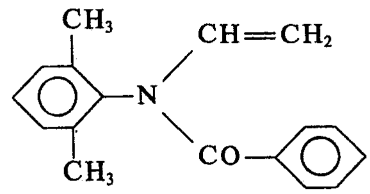

(Verfahren a)

Zu einer Lösung von 140 g (1 Mol) Benzoylchlorid in 1000 ml Toluol werden bei 0° C unter Rühren 147,2 g (1 Mol) N-Ethyliden-2,6-dimethylanilin eingetropft. Man läßt 2 Stunden bei 20° C nachrühren und tropft dann 101 g (1 Mol) Triethylamin zu. Nach weiterem Nachrühren (2 Stunden) saugt man vom ausgefallenen Triethylaminhydrochlorid ab, wäscht die organische Phase mehrfach mit Wasser und dampft im Vakuum ein. Der Rückstand wird aus Ligroin fraktioniert kristallisiert. Man erhält 117 g (46,6% der Theorie) N-Benzoyl-N-vinyl-2,6-dimethylanilin in Form farbloser Kristalle vom Schmelzpunkt 90° C.

Beispiel 4

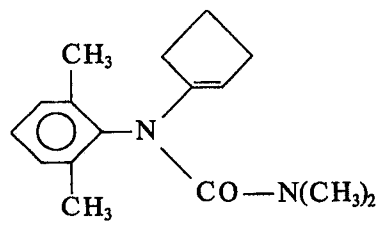

(Verfahren a)

In eine siedende Lösung von 187 g (1 Mol) N-Cyclopentyliden-2,6-dimethylanilin in 1000 ml Tetrachlorkohlenstoff werden 107 g (1 Mol) N,N-Dimethylcarbamidsäurechlorid eingetropft. Man erhitzt weitere 4 Stunden unter Rückfluß und gibt dann bei 20° C 101 g (1 Mol) Triethylamin zu. Man rührt weitere 3 Stunden nach, filtriert vom ausgefallenen Triethylamin-hydrochlorid ab, wäscht das Filtrat mehrfach mit Wasser und dampft dann im Vakuum zur Trockne ein. Man erhält 70 g (32,6% der Theorie) N-(1-Cyclopentenyl)-N-dimethylcarbamoyl-2,6-dimethylanilin in Form eines braunen, zähflüssigen Öls.

Beispiel 5

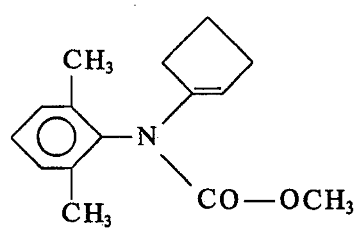

(Verfahren a)

In einer Lösung von 95 g (1 Mol) Chlorameisensäuremethylester in 500 ml Toluol werden bei 20° C unter Rühren 187 g (1 Mol) N-Cyclopentyliden-2,6-dimethylanilin getropft. Man läßt 2 Stunden bei 20° C nachrühren und gibt dann bei 20° C 101 g (1 Mol) Triethylamin zu. Anschließend wird noch 2 Stunden gerührt, dann in 1000 ml Wasser gegossen und mehrfach mit Toluol extrahiert. Die vereinigten Extrakte werden im Vakuum eingedampft und der Rückstand aus Ligroin umkristallisiert. Man erhält 127,5 g (52% der Theorie) N-(1-Cyclopentenyl)-N-methoxycarbonyl-2,6-dimethylanilin in Form farbloser Kristalle vom Schmelzpunkt 91° C.

Beispiel 6

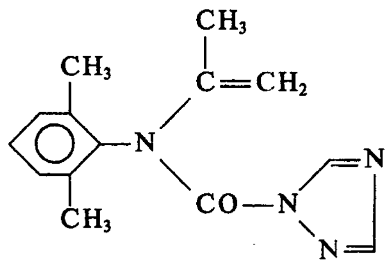

(Verfahren b)

Zu einem Gemisch aus 69 g (1 Mol) 1,2,4-Triazol und 138 g (1 Mol) Kaliumcarbonat in 500 ml Dimethylformamid werden bei 20°C 233 g (1 Mol) N-Chlorcarbonyl-N-(1-methylvinyl)-2,6-dimethylanilin, gelöst in 100 ml Dimethylformamid, getropft. Man rührt 2 Stunden bei 80°C nach und gießt dann auf 2 kg Eis. Der dabei ausfallende Feststoff wird abfiltriert und aus Petrolether umkristallisiert. Man erhält 102 g (79,6% der Theorie) N-(1-Methylvinyl)-N-(1,2,4-triazol-1-yl-carbonyl)-2,6-dimethylanilin in Form farbloser Kristalle vom Schmelzpunkt 65°C.

Herstellung des Ausgangsproduktes

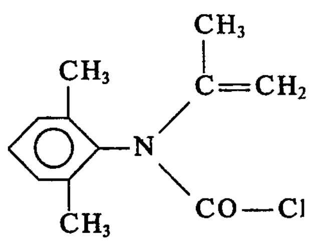

In eine Lösung von 161,2 g (1 Mol) N-Isopropyliden-2,6-dimethylanilin in 500 ml Toluol werden bei 0°C unter Rühren 49,5 g (0,5 Mol) Phosgen eingeleitet. Anschließend rührt man 2 Stunden bei 20°C nach, vertreibt überschüssiges Phosgen durch Einleiten von Stickstoff und saugt dann vom ausgefallenen Niederschlag ab. Das Filtrat wird im Vakuum eingedampft, der Eindampfrückstand im Hochvakuum fraktioniert destilliert. Man erhält 107 g (96% der Theorie) N-Chlorcarbonyl-N-(1-methylvinyl)-2,6-dimethylanilin in Form eines farblosen Öls vom Siedepunkt 85°C/0,3 mbar.

Beispiel 7

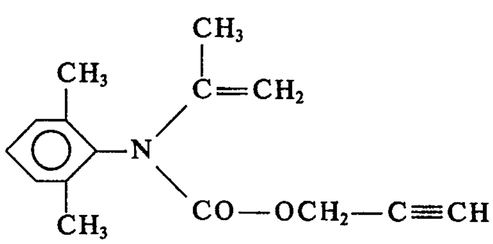

(Verfahren b)

2,4 g (0,1 Mol) Natriumhydrid werden unter Kühlung in 50 ml Propargylalkohol eingetragen. Nach Beendigung der Wasserstoffentwicklung werden bei 20°C 22,4 g (0,1 Mol) N-Chlorcarbonyl-N-(1-methylvinyl)-2,6-dimethylanilin zugetropft. Man läßt 2 Stunden bei 20°C nachrühren, gießt dann die Mischung in 500 ml Wasser und extrahiert mehrfach mit Essigsäureethylester. Die vereinigten Extrakte werden im Vakuum eingeengt. Der Rückstand wird im Vakuum fraktioniert destilliert. Man erhält 18,2 g (74,8% der Theorie) N-(1-Methylvinyl)-N-propargyloxycarbonyl-2,6-dimethylanilin in Form eines farblosen Öls vom Siedepunkt 72—76°C/0,2 mbar.

Beispiel 8

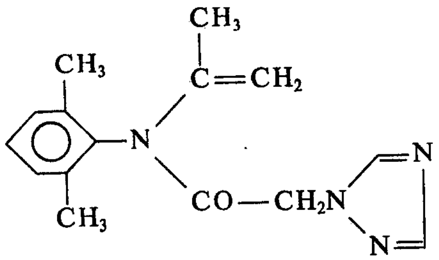

(Verfahren c)

Zu einem Gemisch aus 69 g (1 Mol) 1,2,4-Triazol und 138 g (1 Mol) Kaliumcarbonat in 500 ml Dimethylformamid werden bei 20° C 119 g (1 Mol) N-Chloracetyl-N-(1-methylvinyl)-2,6-dimethylanilin, gelöst in 100 ml Dimethylfiormamid, getropft. Man rührt 2 Stunden bei 80° C nach und gießt die Mischung dann auf 1 kg Eis. Der dabei ausgefallene Niederschlag wird abfiltriert und aus Ligroin umkristallisiert. Man erhält 90 g (66,7% der Theorie) N-(1-Methylvinyl)-N-(1,2,4-triazol-1-yl-acetyl)-2,6-dimethylanilin in Form farbloser Kristalle vom Schmelzpunkt 65° C.

Herstellung des Ausgangsproduktes

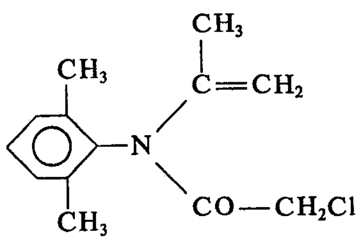

Zu einer Lösung von 113 g (1 Mol) Chloracetylchlorid in 100 ml Toluol werden 322,5 g (2 Mol) N-Isopropyliden-2,6-dimethylanilin getropft wobei die Temperatur bei 0° C gehalten wird. Anschließend wird noch 3 Stunden bei Raumtemperatur nachgerührt. Man filtriert vom ausgefallenen Niederschlag (N-Isopropyliden-2,6-dimethylamin-hydrochlorid) ab und wäscht das Filtrat mehrfach mit Bicarbonatlösung, dann mit Wasser. Die organische Phase wird über Natriumsulfat getrocknet und im Vakuum eingedampft. Der Rückstand wird im Vakuum fraktioniert destilliert. Man erhält 180,6 g (76% der Theorie) N-Chloracetyl-N-(1-methylvinyl)-2,6-dimethylanilin als farbloses Öl vom Siedepunkt 98—106° C/0,15 mbar.

Beispiel 9

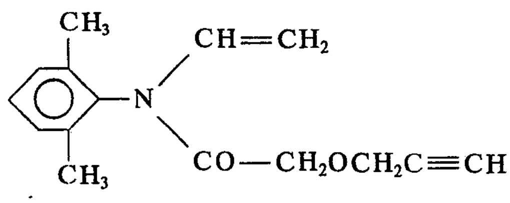

(Verfahren c)

2,3 g (0,1 Mol) Natrium werden zu 50 ml Propargylalkohol gegeben. Nach Beendigung der Wasserstoffentwicklung werden 22,4 g (0,1 Mol) N-Chloracetyl-N-vinyl-2,6-dimethylanilin, gelöst in 50 ml Propargylalkohol, zugetropft. Anschließend wird 3 Stunden bei 20° C nachgerührt. Man gießt die Mischung in Methanol, verdünnt mit 1000 ml Wasser und extrahiert dann mehrfach mit Essigester. Die vereinigten Extrakte werden im Vakuum eingedampft und der Rückstand aus Petrolether umkristallisiert. Man erhält 13 g (53,5% der Theorie) N-Propargyloxyacetyl-N-vinyl-2,6-dimethylanilin in Form farbloser Kristalle vom Schmelzpunkt 72° C.

Beispiel 10

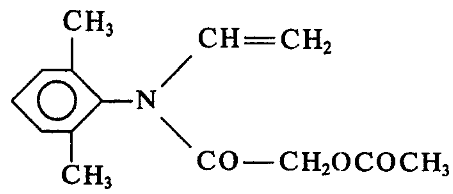

(Verfahren c)

22 g (0,1 Mol) N-Chloracetyl-N-vinyl-2,6-dimethylanilin, gelöst in 50 ml Dimethylformamid werden mit 16,4 g (0,2 Mol) Natriumacetat versetzt und 1 Stunde auf 80° C erhitzt. Nach dem Abkühlen wird auf 500 g Eis gegossen, der dabei ausgefallene Niederschlag abfiltriert und aus Petrolether umkristallisiert. Man erhält 21 g (85% der Theorie) N-Acetoxyacetyl-N-vinyl-2,6-dimethylanilin in Form farbloser Kristalle vom Schmelzpunkt 106° C.

Beispiel 11

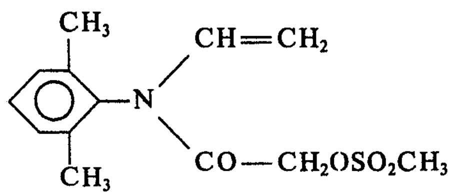

(Verfahren d)

Zu einem Gemisch aus 20,5 g (0,1 Mol) N-Hydroxyacetyl-N-vinyl-2,6-dimethylanilin und 40,4 g (0,4 Mol) Triethylamin in 250 ml Methylenchlorid werden bei 20° C 22,8 g (0,2 Mol) Methansulfonsäurechlorid getropft. Man rührt 2 Stunden bei Raumtemperatur nach und filtriert dann vom ausgefallenen Triethylaminhydrochlorid ab. Das Filtrat wird zweimal mit je 200 ml wäßriger Pyridinlösung (10%ig), dann zweimal mit je 200 ml 20%iger Salzsäure, zuletzt mehrfach mit Wasser gewaschen. Die organische Phase wird über Natriumsulfat getrocknet und im Vakuum eingedampft. Man erhält 26,7 g (94% der Theorie) N-Mesyloxyacetyl-N-vinyl-2,6-dimethylanilin in Form eines farblosen Öls.

Herstellung des Ausgangsproduktes

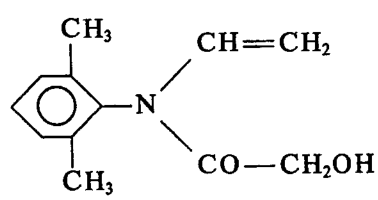

Zu einer Lösung von 24,7 g (0,1 Mol) N-Acetoxyacetyl-N-vinyl-2,6-dimethylanilin (Bsp. 10) in 500 ml Methanol werden bei 20° C 11,2 g (0,2 Mol) Kaliumhydroxid, gelöst in 250 ml Methanol, getropft. Man rührt 5 Stunden bei 20° C. Dann wird im Vakuum das Lösungsmittel abgedampft, der Rückstand in Wasser aufgenommen und mehrfach mit Essigester extrahiert. Die vereinigten Extrakte werden im Vakuum eingedampft, der Eindampfrückstand aus Petrolether umkristallisiert. Man erhält 16,8 g (81,6% der Theorie) N-Hydroxyacetyl-N-vinyl-2,6-dimethylanilin in Form farbloser Kristalle vom Schmelzpunkt 90° C.

Beispiel 12

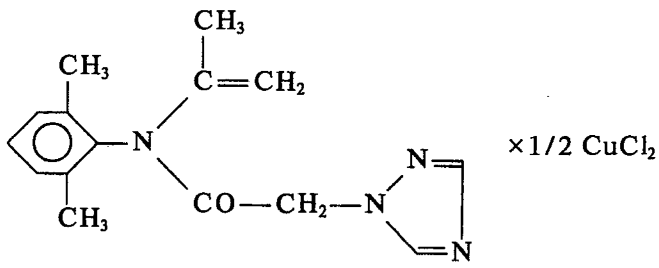

(Metallsalz-Komplex-Bildung)

Zu einer Lösung von 5,4 g (0,02 Mol) N-(1-Methylvinyl)-N-(1,2,4-triazol-1-yl-acetyl)-2,6-dimethyl-anilin in 50 ml Methanol werden bei 20°C 1,7 g (0,01 Mol) Kupfer-II-chloriddihydrat, gelöst in 50 ml Methanol, gegeben. Die Lösung wird im Vakuum eingedampft und der Rückstand mehrfach durch Aufnehmen in Toluol und anschließendes Eindampfen azeotrop entwässert. Der Rückstand wird aus Tetrachlorkohlenstoff umkristallisiert. Man erhält 5 g (74,1% der Theorie) N-(1-Methylvinyl)-N-(1,2,4-triazol-1-yl-acetyl)-2,6-dimethylanilin-kupfer-II-chlorid in Form schwach blau gefärbter Kristalle vom Schmelzpunkt 120°C.

Beispiel 13

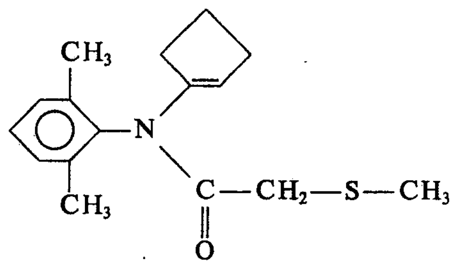

(Verfahren c)

Zu einer Lösung von 9,2 g (0,4 Mol) Natrium in 200 ml Methanol wird bei 20°C eine Lösung von 12 g (0,4 Mol) Methylmercaptan in 50 ml Methanol getropft. In diese Lösung werden 58 g (0,22 Mol) N-(1-Cyclopentenyl)-N-(2,6-dimethyl)-chloracetamid unter Rühren eingetropft. Durch Kühlung wird die Reaktion bei 30°C gehalten. Man rührt 2 Stunden bei Raumtemperatur nach und erhitzt anschließend eine Stunde am Rückfluß. Danach wird vom ausgefallenen Natriumchlorid abfiltriert, das Filtrat im Vakuum eingedampft. Der Eindampfrückstand wird im Essigsäureethylester aufgenommen, mehrfach mit Wasser gewaschen, über Natriumsulfat getrocknet und danach erneut im Vakuum eingedampft.

Man erhält 57 g eines gelben Öles, das zur weiteren Reinigung im Hochvakuum fraktioniert destilliert wird. Man erhält 38 g (62% der Theorie) N-(1-Cyclopentenyl)-N-methylthioacetyl-2,6-dimethylanilin in Form eines farblosen Öles vom Siedepunkt 129–30°C bei 0,1 mbar.

Herstellung des Ausgangsproduktes

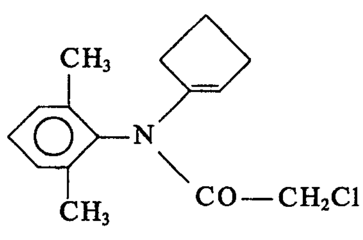

(Verfahren a)

Zu einer Lösung von 22,6 g (0,2 Mol) Chloracetylchlorid in 20 ml Toluol werden bei 20°C 37,4 g (0,2 Mol) N-Cyclopentyliden-2,6-dimethylanilin getropft. Man läßt 2 Stunden bei 20°C nachrühren und tropft dann bei 20°C 20,2 g (0,2 Mol) Triethylamin zu. Man rührt 2 Stunden bei 20°C nach und filtriert ab. Das Filtrat wird mehrfach mit 10%iger wäßriger Bicarbonatlösung, dann mit Wasser gewaschen, über Natriumsulfat getrocknet und im Vakuum eingedampft. Der Eindampfrückstand wird im Hochvakuum

fraktioniert destilliert. Man erhält 41,2 g (78% der Theorie) N-(1-Cyclopentenyl)-N-(2,6-dimethyl)-chloracetamid in Form eines gelben Öles vom Siedepunkt 127° C/0,2 mbar.

### Beispiel 14

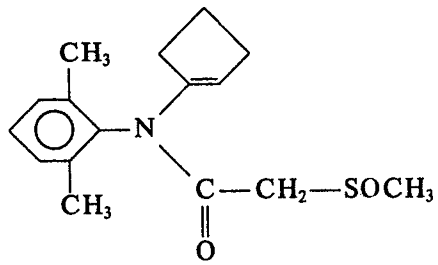

(Verfahren e)

Zu einer Lösung von 14,6 g (0,04 Mol) N-(1-Cyclopentenyl)-N-methylthioacetyl-2,6-dimethylanilin in 70 ml Dichlormethan wird bei 0° C eine Lösung von 8,1 g (0,04 Mol) 85%iger 3-Chlor-perbenzoesäure in 150 ml Dichlormethan getropft. Man rührt 1 Stunde bei 0° C, dann 6 Stunden bei 20° C. Anschließend wird mehrfach mit gesättigter wäßriger Natriumcarbonatlösung, dann mit Wasser gewaschen, die organische Phase über Natriumsulfat getrocknet und im Vakuum eingedampft. Man erhält einen öligen Rückstand, der zur weiteren Reinigung an Kieselgel (Laufmittel: Chloroform) fraktioniert chromatographiert wird. Man erhält 6,2 g (53% der Theorie) N-(1-Cyclopentenyl)-N-methylsulfinylacetyl--2,6-dimethylanilin in Form farbloser Kristalle vom Schmelzpunkt 117° C (aus Ligroin).

### Beispiel 15

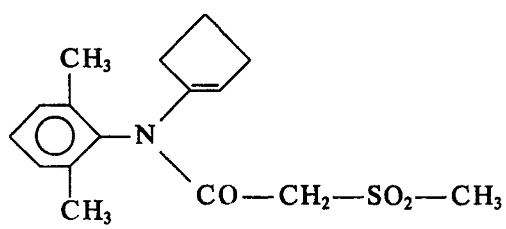

(Verfahren e)

Zu einer Lösung von 16,5 g (0,06 Mol) N-(1-Cyclopentenyl)-N methylthioacetyl-2,6-dimethylanilin in 150 ml Dichlormethan wird bei 0° C eine Lösung von 24,4 g (0,12 Mol) 85%iger 3-Chlor-perbenzoesäure in 350 ml Dichlormethan getropft. Man rührt 1 Stunde bei 0° C, dann 16 Stunden bei 20° C. Anschließend wird die Reaktionslösung mehrfach mit gesättigter wäßriger Natriumcarbonatlösung, dann mit Wasser gewaschen. Die organische Phase wird über Natriumsulfat getrocknet und im Vakuum eingedampft. Man erhält 13,3 g (72% der Theorie) N-(1-Cyclopentenyl)-N-methylsulfonylacetyl-2,6-dimethylanilin in Form eines farblosen Öls.

In analoger Weise und entsprechend den angegebenen Verfahrensvarianten können die in der folgenden Tabelle 1 aufgeführten Verbindungen der allgemeinen Formel (I) erhalten werden:

Tabelle 1

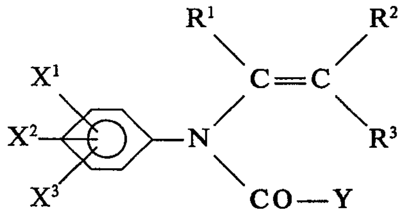

(I)

| Bsp. Nr. | $X^1$ | $X^2$ | $X^3$ | $-CR^1=CR^2R^3$ | Y | Physikalische Konstante |
|---|---|---|---|---|---|---|
| 16 | 2-$CH_3$ | 6-$CH_3$ | H | $-CH=CH_2$ | $-CH_2OCH_3$ | Kp.: 85°C/0,4 mbar |
| 17 | 2-$CH_3$ | 6-$CH_3$ | | $-CH=CH-CH=CH_2$ | $-CH_2OCH_3$ | Fp.: 100°C |
| 18 | 2-$CH_3$ | 6-$CH_3$ | | $-CH=$ 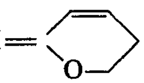 | $-CH_2OCH_3$ | Fp.: 75°C |
| 19 | 2-$CH_3$ | 6-$CH_3$ | | 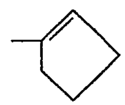 | $-CH_2OCH_3$ | Öl |
| 20 | 2-$CH_3$ | 6-$CH_3$ | | $-CH=C(CH_3)_3$ | $-CH_2OCH_3$ | Kp.: 96-98°C/0,25 mbar |
| 21 | 2-$CH_3$ | 6-$CH_3$ | | $-CH=CH_2$ | $-OCH_3$ | Fp.: 64°C |
| 22 | 2-$CH_3$ | 6-$CH_3$ | | $-CH=CH-CH=CH_2$ | $-OCH_3$ | Fp.: 109°C |
| 23 | 2-$CH_3$ | 6-$CH_3$ | | $-C(CH_3)=CH_2$ | $-OCH_3$ | Fp.: 60°C |
| 24 | 2-$CH_3$ | 6-$CH_3$ | | $-C(CH_3)=CH_2$ | 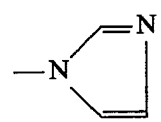 | Fp.: 88°C |
| 25 | 2-$CH_3$ | 6-$CH_3$ | | $-C(CH_3)=CH_2$ | 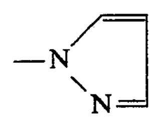 | Fp.: 62°C |
| 26 | 2-$CH_3$ | 6-$CH_3$ | H | 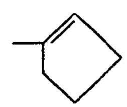 | 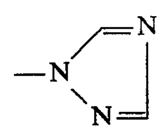 | Fp.: 140°C |

Fortsetzung

| Bsp. Nr. | $X^1$ | $X^2$ | $X^3$ | $-CR^1{=}CR^2R^3$ | Y | Physikalische Konstante |
|---|---|---|---|---|---|---|
| 27 | 2-$CH_3$ | 6-$CH_3$ | H | 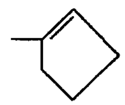 | 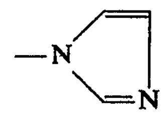 | Fp.: 116°C |
| 28 | 2-$CH_3$ | 6-$CH_3$ | H | $-C(CH_3){=}CH_2$ | 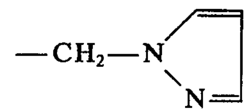 | Fp.: 55°C |
| 29 | 2-$CH_3$ | 6-$CH_3$ | H | $-C(CH_3){=}CH_2$ | 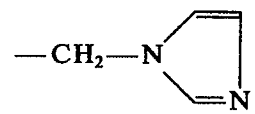 | Fp.: 56°C |
| 30 | 2-$CH_3$ | 6-$CH_3$ | H | $-CH{=}CH_2$ | 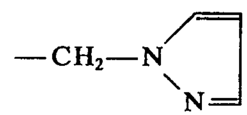 | Kp.: 147°C/0,6 mbar |
| 31 | 2-$CH_3$ | 6-$CH_3$ | H | $-CH{=}CH_2$ | 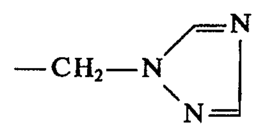 | Fp.: 122°C |
| 32 | 2-$CH_3$ | 6-$CH_3$ | H | $-CH{=}CH_2$ | 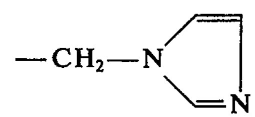 | Fp.: 85°C |
| 33 | 2-$CH_3$ | 6-$CH_3$ | H | 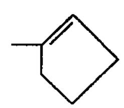 | 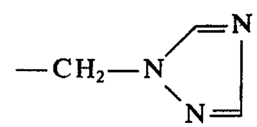 | Fp.: 85°C |
| 34 | 2-$CH_3$ | 6-$CH_3$ | H | 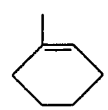 | 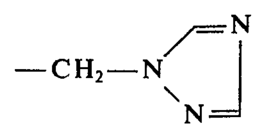 | Fp.: 88°C |

Fortsetzung

| Bsp. Nr. | $X^1$ | $X^2$ | $X^3$ | $—CR^1{=}CR^2R^3$ | Y | Physikalische Konstante |
|---|---|---|---|---|---|---|
| 35 | 2-$CH_3$ | 6-$CH_3$ | H | 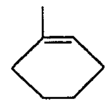 | —$CH_2$—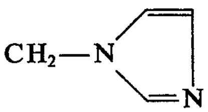 | Fp.: 83°C |
| 36 | 2-$CH_3$ | 6-$CH_3$ | H | 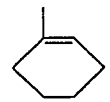 | —$CH_2$—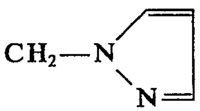 | Öl |
| 37 | 2-$C_2H_5$ | 6-$C_2H_5$ | H | $—CH{=}C(CH_3)_2$ | —$CH_2$—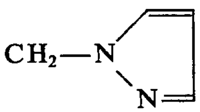 | $n_D^{20}$: 1,5510 |
| 38 | 2-$C_2H_5$ | 6-$C_2H_5$ | H | $—CH{=}C(CH_3)_2$ | —$CH_2$—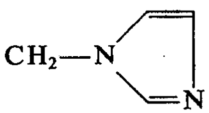 | $n_D^{20}$: 1,5529 |
| 39 | 2-$CH_3$ | 6-$CH_3$ | H | $—C(CH_3){=}CH_2$ | —$CH_2$—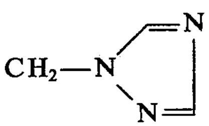 | Fp.: 155°C/(×1/2 $ZnCl_2$) |
| 40 | 2-$CH_3$ | 6-$CH_3$ | H | $—C(CH_3){=}CH_2$ | —$CH_2$—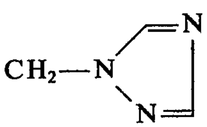 | Fp.: 205°C/(×1/2 $MnCl_2$) |
| 41 | 2-$CH_3$ | 6-$CH_3$ | H | $—C(CH_3){=}CH_2$ | $—CH_2OCH_2C{\equiv}CH$ | Öl |
| 42 | 2-$CH_3$ | 6-$CH_3$ | H | $—C(CH_3){=}CH_2$ | $—CH_2OCOCH_3$ | Öl |
| 43 | 2-$CH_3$ | 6-$CH_3$ | H | $—C(CH_3){=}CH_2$ | $—CH_2OSO_2CH_3$ | Öl |
| 44 | 2-$CH_3$ | 6-$CH_3$ | H | $—C(CH_3){=}CH_2$ | $—CHCl_2$ | Fp.: 41°C |

Fortsetzung

| Bsp. Nr. | $X^1$ | $X^2$ | $X^3$ | $-CR^1=CR^2R^3$ | Y | Physikalische Konstante |
|---|---|---|---|---|---|---|
| 45 | $2\text{-}CH_3$ | $6\text{-}CH_3$ | H | $-CH=CH-CH=CH$<br>$\qquad\qquad\qquad\quad|$<br>$\qquad\qquad\qquad\quad CH=CH_2$ | $-CH_2OCH_3$ | Öl |
| 46 | $2\text{-}CH_3$ | $6\text{-}CH_3$ | H | $-C(CH_3)=CH-OCH_3$ | $-CH_2-$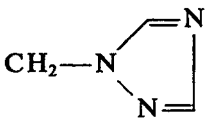 | Öl |
| 47 | $2\text{-}CH_3$ | $6\text{-}CH_3$ | H | $-CH=C(CH_3)_2$ | $-CH_2-$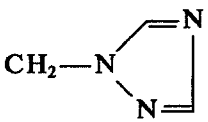 | Öl |
| 48 | $2\text{-}CH_3$ | $6\text{-}CH_3$ | H | $-C(CH_3)=CH_2$ | $-O-CH_2-CH=CH_2$ | Kp.: 90°C/0,2 mbar |
| 49 | $2\text{-}CH_3$ | $6\text{-}CH_3$ | H | $-C(CH_3)=CH_2$ | $-NHCH_2-CH=CH_2$ | Kp.: 108-111°C/0,35 mbar |
| 50 | $2\text{-}CH_3$ | $6\text{-}CH_3$ | H | $-C(CH_3)=CH_2$ | $-O-$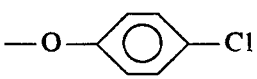 | Fp.: 46°C |
| 51 | $2\text{-}CH_3$ | $6\text{-}CH_3$ | H | $-C(CH_3)=CH_2$ | $-OCH(CH_3)_2$ | Öl |
| 52 | $2\text{-}CH_3$ | $6\text{-}CH_3$ | H | $-C(CH_3)=CH_2$ | $-O-$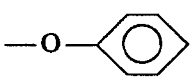 | Öl |
| 53 | $2\text{-}CH_3$ | $6\text{-}CH_3$ | H | 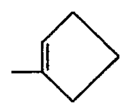 | $-CH_2-$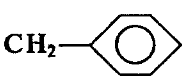 | Öl |
| 54 | $2\text{-}CH_3$ | $6\text{-}CH_3$ | H | $-CH=CH_2$ | $-CH_2-O-$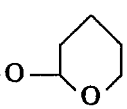 | Kp.: 135°C/0,3 mbar |
| 55 | $2\text{-}CH_3$ | $6\text{-}CH_3$ | H | $-CH=CH-C_2H_5$ | $-CH_2OCH_3$ | Öl |

Fortsetzung

| Bsp. Nr. | $X^1$ | $X^2$ | $X^3$ | $-CR^1{=}CR^2R^3$ | Y | Physikalische Konstante |
|---|---|---|---|---|---|---|
| 56 | 2-$CH_3$ | 6-$CH_3$ | H | $-CH{=}CH-C_2H_5$ | 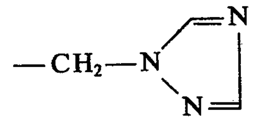 | Fp.: 98°C |
| 57 | 2-$CH_3$ | 6-$CH_3$ | H | $-CH{=}CH-CH{=}CH_2$ | 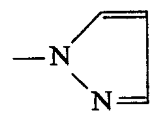 | Fp.: 116°C |
| 58 | 2-$CH_3$ | 6-$CH_3$ | H | $-CH{=}CH-CH{=}CH_2$ | 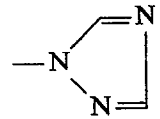 | Fp.: 112°C |
| 59 | 2-$CH_3$ | 6-$CH_3$ | H | $-CH{=}CH-CH{=}CH_2$ | 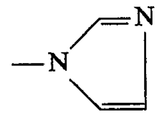 | Fp.: 106°C |
| 60 | 2-$CH_3$ | 6-$CH_3$ | H | $-CH{=}CH-COCH_3$ | $-CH_2OCH_3$ | Fp.: 76–80°C |
| 61 | 2-$CH_3$ | 6-$CH_3$ | H | 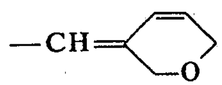 | 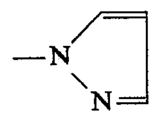 | Fp.: 127°C |
| 62 | 2-$CH_3$ | 6-$CH_3$ | H | 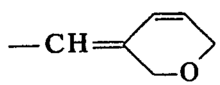 | 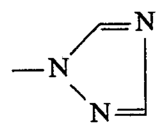 | Fp.: 161°C |
| 63 | 2-$CH_3$ | 6-$CH_3$ | H | 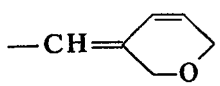 | 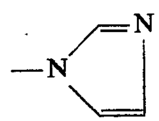 | Fp.: 186°C |

Fortsetzung

| Bsp. Nr. | $X^1$ | $X^2$ | $X^3$ | $-CR^1{=}CR^2R^3$ | Y | Physikalische Konstante |
|---|---|---|---|---|---|---|
| 64 | $2\text{-}CH_3$ | $6\text{-}CH_3$ | H | $-C(CH_3){=}CH_2$ | 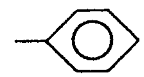 | Fp.: 57°C |
| 65 | $2\text{-}CH_3$ | $6\text{-}CH_3$ | H | $-C(CH_3){=}CH_2$ | 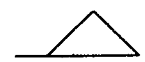 | Fp.: 45°C |
| 66 | $2\text{-}CH_3$ | $6\text{-}CH_3$ | H | $-C(CH_3){=}CH_2$ | $-CH_2SCN$ | Öl |
| 67 | $2\text{-}CH_3$ | $6\text{-}CH_3$ | H | $-C(CH_3){=}CH_2$ | $-CH_2SCH_3$ | Öl |
| 68 | $2\text{-}CH_3$ | $6\text{-}CH_3$ | H | $-C(CH_3){=}CH_2$ | $-CH_2SOCH_3$ | Fp.: 78°C |
| 69 | $2\text{-}CH_3$ | $6\text{-}CH_3$ | H | $-C(CH_3){=}CH_2$ | $-CH_2SO_2CH_3$ | Fp.: 83°C |
| 70 | $2\text{-}CH_3$ | $6\text{-}CH_3$ | H | $-C(C_3H_7\text{-}i){=}CH_2$ | $-CH_2OCH_3$ | Kp.: 150°C/0,8 mbar |
| 71 | $2\text{-}CH_3$ | $6\text{-}CH_3$ | H | $-C(C_3H_7\text{-}i){=}CH_2$ | $-CH_2-N-$ 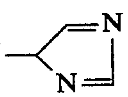 | Öl |
| 72 | $2\text{-}CH_3$ | $6\text{-}CH_3$ | H | $-C(CH_3){=}CH_2$ | 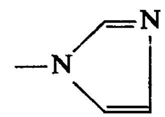 | Öl |
| 73 | $2\text{-}CH_3$ | $6\text{-}CH_3$ | H | 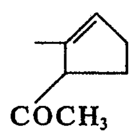 | $-CH_2OCH_3$ | Öl |
| 74 | 2-Cl | $6\text{-}CH_3$ | H | $-C(CH_3){=}CH-C(CH_3){=}CH_2$ | $-CH_2OCH_3$ | Öl |
| 75 | 2-Cl | $6\text{-}CH_3$ | H | $-C(CH_3){=}CH-C(CH_3){=}CH_2$ | 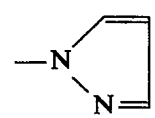 | Öl |

Fortsetzung

| Bsp. Nr. | $X^1$ | $X^2$ | $X^3$ | $-CR^1{=}CR^2R^3$ | Y | Physikalische Konstante |
|---|---|---|---|---|---|---|
| 76 | 2-Cl | 6-$CH_3$ | H | $-C(CH_3){=}CH-C(CH_3){=}CH_2$ | 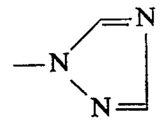 | Öl |
| 77 | 2-Cl | 6-$CH_3$ | H | $-C(CH_3){=}CH-C(CH_3){=}CH_2$ | 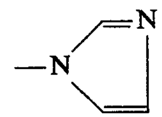 | Öl |
| 78 | 2-Cl | 6-$CH_3$ | H | 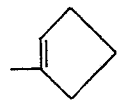 | $-CH_2OCH_3$ | Öl |
| 79 | 2-Cl | 6-$CH_3$ | H | 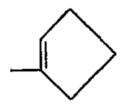 | 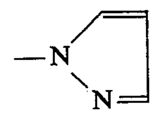 | Fp.: 122°C |
| 80 | 2-Cl | 6-$CH_3$ | H | 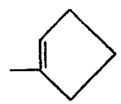 | 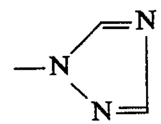 | Fp.: 132°C |
| 81 | 2-Cl | 6-$CH_3$ | H | 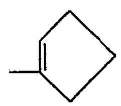 | 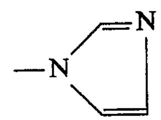 | Fp.: 106°C |
| 82 | 2-Cl | 6-$CH_3$ | H | 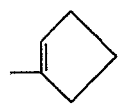 | 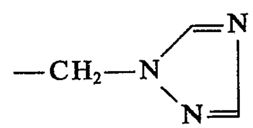 | Öl |
| 83 | 2-$CH_3$ | 4-$CH_3$ | 6-$CH_3$ | $-C(CH_3){=}CH_2$ | $-CH_2OCH_3$ | Kp.: 115°C/0,4 mbar |
| 84 | 2-$CH_3$ | 4-$CH_3$ | 6-$CH_3$ | $-C(CH_3){=}CH_2$ | 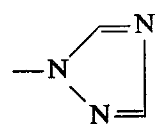 | Öl |

Fortsetzung

| Bsp. Nr. | $X^1$ | $X^2$ | $X^3$ | $-CR^1{=}CR^2R^3$ | Y | Physikalische Konstante |
|---|---|---|---|---|---|---|
| 85 | 2-$CH_3$ | 6-$CH_3$ | H | $-C(CH_3){=}CH-C(CH_3){=}CH_2$ | 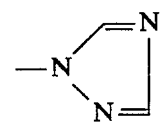 | Öl |
| 86 | 2-$CH_2$ | 6-$CH_3$ | H | $-C(CH_3){=}CH-C(CH_3){=}CH_2$ | 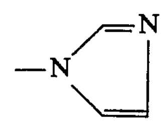 | Öl |
| 87 | 2-$CH_3$ | 6-$CH_3$ | H | $-CH{=}CH_2$ | 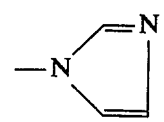 | Öl |

## Anwendungsbeispiele

In den nachfolgenden Anwendungsbeispielen werden die nachstehend angegebenen Verbindungen als Vergleichssubstanzen eingesetzt:

(A) 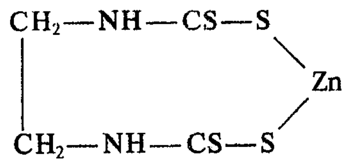

(B) 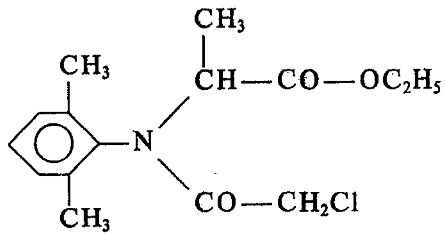

(C) 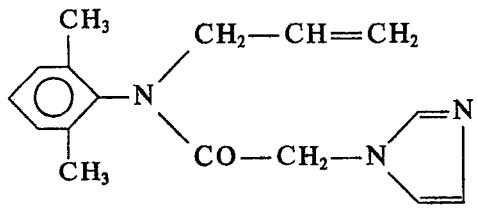

(D) 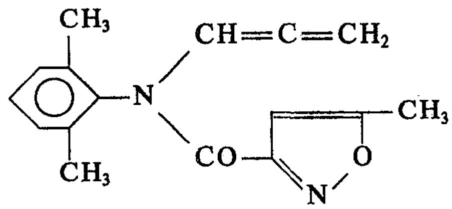

(E) 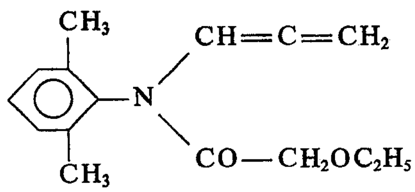

### Beispiel A

Phytophthora-Test (Tomate)/protektiv

| | |
|---|---|
| Lösungsmittel: | 4,7 Gewichtsteile Aceton |
| Emulgator: | 0,3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele: 1, 8, 16, 30, 31.

Beispiel B

Phytophthora-Test (Tomate)/systemisch

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf systemische Eigenschaften wird die Wirkstoffzubereitung auf Einheitserde gegossen, in der sich junge versuchsbereite Pflanzen befinden. 3 Tage nach der Behandlung werden die Pflanzen mit einer wäßrigen Sporensuspension von Phytophthora infestans inokuliert.

Die Pflanzen werden in einer Inkubationskabine mit 100% relativer Luftfeuchtigkeit und ca. 20°C aufgestellt.

3 Tage nach der Inokulation erfolgt die Auswertung.

Eine deutliche Überlegenheit in der Wirksamkeit gegenüber dem Stand der Technik zeigen in diesem Test z. B. die Verbindungen gemäß folgender Herstellungsbeispiele: 1, 8 und 9.

**Patentansprüche**

1. N-(1-Alkenyl)-carbonsäureanilide der allgemeinen Formel (I)

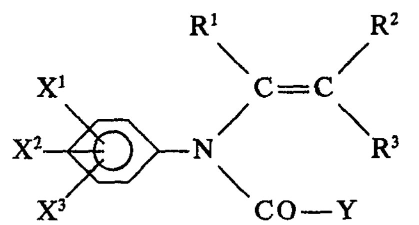

(I)

in welcher

$R^1$ für Wasserstoff und Alkyl steht,
$R^2$ für Wasserstoff, Alkyl, Alkoxy, Alkoxyalkyl, Hydroxyalkyl oder Benzyl steht,
$R^3$ für Wasserstoff, Alkyl, Alkenyl, Alkoxy, Alkoxyalkyl, Alkylcarbonyl, Alkylcarbonylalkyl, Alkoxycarbonyl oder Alkoxycarbonylalkyl steht, oder
$R^1$ und $R^2$ gemeinsam mit der C=C-Doppelbindung für einen gegebenenfalls ein- oder mehrfach durch Alkyl mit 1 bis 4 Kohlenstoffatomen, Halogen und Hydroxy substituierten, einfach oder mehrfach ungesättigten Ring stehen, der auch Heteroatome enthalten kann, oder
$R^2$ und $R^3$ gemeinsam mit dem angrenzenden Kohlenstoffatom für einen gegebenenfalls ein- oder mehrfach durch Alkyl mit 1 bis 4 Kohlenstoffatomen, Halogen und Hydroxy substituierten, gesättigten oder ungesättigten Ring stehen, der auch Heteroatome enthalten kann,
$X^1$, $X^2$ und $X^3$ unabhängig voneinander für Wasserstoff, Alkyl oder Halogen stehen,
Y für Furyl, Tetrahydrofuryl, Thienyl, Tetrahydrothienyl; gegebenenfalls durch Halogen, Alkyl, Alkoxy und Alkylthio mit jeweils 1 bis 4 Kohlenstoffatomen, Halogenalkyl, Halogenalkoxy und Halogenalkylthio mit jeweils 1 bis 4 Kohlenstoff- und 1 bis 5 gleichen oder verschiedenen Halogenatomen, Nitro, Cyano sowie gegebenenfalls durch Halogen und Alkyl mit 1 bis 4 Kohlenstoffatomen substituiertes Phenyl, substituiertes Phenyl; gegebenenfalls durch Alkyl substituiertes Isoxazolyl; 1,2,4-Triazol-1-yl, Imidazol-1-yl und Pyrazol-1-yl, gegebenenfalls durch Cyano oder Thiocyano substituiertes Alkyl, Alkenyl und Alkinyl; gegebenenfalls durch Alkyl mit 1 bis 4 Kohlenstoffatomen, Alkenyl mit 2 bis 4 Kohlenstoffatomen, Halogenalkyl mit 1 bis 4 Kohlenstoff- und bis zu 5 gleichen oder verschiedenen Halogenatomen, Halogenalkenyl mit 2 bis 4 Kohlenstoff- und bis zu 5 gleichen oder verschiedenen Halogenatomen, Halogen, Alkoxy mit 1 bis 4 Kohlenstoffatomen sowie eine zwei- bis fünfgliedrige Methylenbrücke substituiertes Cycloalkyl oder Cycloalkenyl; Dihalogenalkyl; sowie die Gruppierungen

$-CH_2Az$ $-CH_2R^9$ $-CH_2OR^8$ $-CH_2OR^9$ $-CH_2SR^8$ $-CH_2SR^9$

$-CH_2-SO_nR^8$ $-CH_2SO_n-R^9$ $-CH_2OSOR^8$ $-CH_2OSOR^9$ $-CH_2OSO_2R^8$

$-CH_2OSO_2R^9$ $-CH_2OCOR^8$ $-CH_2OCOR^9$ $-OR^8$ $-OR^9$ $-SR^8$

$-SR^9$ $-NR^{10}R^{11}$ $-CH_2-O-$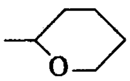

steht, wobei

$R^8$ für gegebenenfalls durch Halogen, Cyano oder Thiocyano substituiertes Alkyl, Alkenyl, Alkinyl und Alkoxyalkyl steht,

$R^9$ für gegebenenfalls durch Halogen, Alkyl, Alkoxy und Alkylthio mit jeweils 1 bis 4 Kohlenstoffatomen, Halogenalkyl, Halogenalkoxy und Halogenalkylthio mit jeweils 1 bis 4 Kohlenstoff- und 1 bis 5 gleichen oder verschiedenen Halogenatomen, Nitro, Cyano sowie gegebenenfalls durch Halogen und Alkyl mit 1 bis 4 Kohlenstoffatomen substituiertes Phenyl substituiertes Phenyl steht,

$R^{10}$ und $R^{11}$ gleich oder verschieden sind und für Wasserstoff, Alkyl, Alkenyl oder gegebenenfalls durch Halogen, Alkyl, Alkoxy und Alkylthio mit jeweils 1 bis 4 Kohlenstoffatomen, Halogenalkyl, Halogenalkoxy und Halogenalkylthio mit jeweils 1 bis 4 Kohlenstoff- und 1 bis 5 gleichen oder verschiedenen Halogenatomen, Nitro, Cyano sowie gegebenenfalls durch Halogen oder Alkyl mit 1 bis 4 Kohlenstoffatomen substituierten Phenyl substituiertes Phenyl stehen,

Az für Pyrazol-1-yl, 1,2,4-Triazol-1-yl und Imidazol-1-yl steht,

n für 1 oder 2 steht,

sowie, wenn

Y für Az bzw. für $-CH_2-Az$ steht, deren physiologisch verträglichen Säureadditionssalze und Metallsalzkomplexe.

2. N-(1-Alkenyl)-carbonsäureanilide der Formel (I), in welcher

$R^1$ für Wasserstoff oder geradkettiges bzw. verzweigtes Alkyl mit 1 bis 4 Kohlenstoffatomen;

$R^2$ für Wasserstoff, geradkettiges oder verzweigtes Alkyl mit 1 bis 4 Kohlenstoffatomen, Alkoxy mit 1 bis 4 Kohlenstoffatomen, Hydroxyalkyl mit 1 bis 4 Kohlenstoffatomen, Alkoxyalkyl mit 1 bis 4 Kohlenstoffatomen im Alkylteil und 1 bis 4 Kohlenstoffatomen im Alkoxyteil oder für Benzyl;

$R^3$ für Wasserstoff, geradkettiges oder verzweigtes Alkyl mit 1 bis 4 Kohlenstoffatomen, Alkoxy mit 1 bis 4 Kohlenstoffatomen, Alkoxyalkyl mit 1 bis 4 Kohlenstoffatomen im Alkylteil und 1 bis 4 Kohlenstoffatomen im Alkoxyteil, Alkylcarbonyl mit 1 bis 4 Kohlenstoffatomen im Alkylteil, Alkylcarbonylalkyl mit 1 bis 4 Kohlenstoffatomen in jedem Alkylteil, Alkoxycarbonyl mit 1 bis 4 Kohlenstoffatomen im Alkoxyteil, Alkoxycarbonylalkyl mit 1 bis 4 Kohlenstoffatomen im Alkoxyteil und 1 bis 4 Kohlenstoffatomen im Alkylteil oder für geradkettiges oder verzweigtes Alkenyl mit 2 bis 6 Kohlenstoffatomen;

oder

$R^1$ und $R^2$ gemeinsam mit der C=C-Doppelbindung für einen gegebenenfalls ein- oder mehrfach substituierten, einfach oder mehrfach ungesättigten, 5- oder 6gliedrigen Ring, der ein oder zwei Heteroatome enthalten kann, wobei als Substituenten genannt seien: Alkyl mit 1 bis 4 Kohlenstoffatomen, Halogen und Hydroxy;

oder

$R^2$ und $R^3$ gemeinsam mit dem angrenzenden Kohlenstoffatom für einen gegebenenfalls ein- oder mehrfach substituierten, gesättigten oder ungesättigten, 5- oder 6gliedrigen Ring, der ein oder zwei Heteroatome enthalten kann, wobei als Substituenten genannt seien: Alkyl mit 1 bis 4 Kohlenstoffatomen, Halogen und Hydroxy;

$X^1$ für Wasserstoff, geradkettiges oder verzweigtes Alkyl mit 1 bis 4 Kohlenstoffatomen, Fluor, Chlor oder Brom;

$X^2$ für Wasserstoff, geradkettiges oder verzweigtes Alkyl mit 1 bis 4 Kohlenstoffatomen, Fluor, Chlor oder Brom; und

$X^3$ für Wasserstoff und geradkettiges Alkyl mit 1 bis 4 Kohlenstoffatomen, Fluor, Chlor oder Brom,

Y für Furyl, Tetrahydrofuryl, Thienyl, Tetrahydrothienyl, für gegebenenfalls substituiertes Phenyl, wobei als Substituenten genannt seien: Halogen, Alkyl, Alkoxy und Alkylthio mit jeweils 1 bis 4 Kohlenstoffatomen, Halogenalkyl, Halogenalkoxy und Halogenalkylthio mit jeweils 1 bis 4 Kohlenstoff- und 1 bis 5 gleichen oder verschiedenen Fluor- und Chloratomen, Nitro, Cyano sowie gegebenenfalls durch Halogen und Alkyl mit 1 bis 4 Kohlenstoffatomen substituiertes Phenyl, für gegebenenfalls durch Methyl oder Ethyl substituiertes Isoxazolyl, für 1,2,4-Triazol-1-yl, Imidazol-1-yl und Pyrazol-1-yl, für gegebenenfalls durch Cyano oder Thiocyano substituiertes Alkyl mit 1 bis 4 Kohlenstoffatomen sowie Alkenyl und Alkinyl mit jeweils 2 bis 4 Kohlenstoffatomen, für gegebenenfalls substituiertes Cycloalkyl mit 3 bis 7 Kohlenstoffatomen oder Cycloalkenyl mit 4 bis 7 Kohlenstoffatomen, wobei als Substituenten infrage kommen: Alkyl mit 1 bis 4 Kohlenstoffatomen, Alkenyl mit 2 bis 4 Kohlenstoffatomen, Halogenalkyl mit 1 bis 4 Kohlenstoff- und bis zu 5 gleichen oder verschiedenen Halogenatomen, Halogenalkenyl mit 2 bis 4 Kohlenstoff- und bis zu 5 gleichen oder verschiedenen Halogenatomen, Halogen, Alkoxy mit 1 bis 4 Kohlenstoffatomen sowie eine zwei- bis fünfgliedrige Methylenbrücke; ferner für Dihalogenalkyl mit 1 bis 2 Kohlenstoffatomen, wobei als Halogenatome Fluor, Chlor und Brom genannt seien; sowie für die Gruppierungen

$-CH_2Az$ $-CH_2R^9$ $-CH_2OR^8$ $-CH_2OR^9$ $-CH_2SR^8$ $-CH_2SR^9$

$-CH_2-SO_nR^8$ $-CH_2SO_n-R^9$ $-CH_2OSOR^8$ $-CH_2OSOR^9$ $-CH_2OSO_2R^8$

$-CH_2OSO_2R^9$ $-CH_2OCOR^8$ $-CH_2OCOR^9$ $-OR^8$ $-OR^9$ $-SR^8$

$-SR^9$ $-NR^{10}R^{11}$ und $-CH_2-O-$ 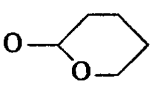

$R^8$ für gegebenenfalls durch Fluor, Chlor oder Brom, Cyano und Thiocyano substituiertes Alkyl mit 1 bis 4 und Alkenyl sowie Alkinyl mit jeweils 2 bis 4 Kohlenstoffatomen und für Alkoxyalkyl mit 1 bis 4 Kohlenstoffatomen in jedem Alkylteil;

$R^9$ für gegebenenfalls substituiertes Phenyl, wobei als Phenylsubstituenten die bei Y bereits genannten infrage kommen;

$R^{10}$ und $R^{11}$ für Wasserstoff, Alkyl mit 1 bis 4 Kohlenstoffatomen, Alkenyl mit 2 bis 4 Kohlenstoffatomen sowie für gegebenenfalls substituiertes Phenyl, wobei als Phenylsubstituenten die bei Y bereits genannten infrage kommen;

Az für Pyrazol-1-yl, 1,2,4-Triazol-1-yl und Imidazol-1-yl; und der Index

n für 1 oder 2.

3. N-(1-Alkenyl)-carbonsäureanilide der Formel (I), in welcher

$R^1$ für Wasserstoff, Methyl, Ethyl oder Isopropyl steht;

$R^2$ für Wasserstoff, Methyl, Ethyl, Isopropyl, Methoxy, Ethoxy, Isopropoxy, Methoxymethyl, Ethoxymethyl, iso-Hydroxypropyl oder Benzyl steht;

$R^3$ für Wasserstoff, Methyl, Ethyl, Isopropyl, Methoxy, Ethoxy, Isopropoxy, Methoxymethyl, Ethoxymethyl, Methylcarbonyl, Ethylcarbonyl, Methylcarbonylmethyl, Methoxycarbonyl, Ethoxycarbonyl, Methoxycarbonylmethyl sowie für geradkettiges oder verzweigtes Alkenyl mit 2 bis 4 Kohlenstoffatomen steht;

oder

$R^1$ und $R^2$ gemeinsam mit der C=C-Doppelbindung für einen gegebenenfalls durch Methyl, Ethyl, Hydroxy und/oder Chlor substituierten, einfach oder mehrfach ungesättigten 5- oder 6gliedrigen Ring stehen, der ein oder zwei Sauerstoff-, Stickstoff- und/oder Schwefel-Atome, enthalten kann;

oder

$R^2$ und $R^3$ gemeinsam mit dem angrenzenden Kohlenstoffatom für einen gegebenenfalls durch Methyl, Ethyl, Hydroxy und/oder Chlor substituierten, gesättigten oder ungesättigten 5- oder 6gliedrigen Ring stehen, der ein oder zwei Sauerstoff-, Stickstoff- oder Schwefel-Atome, enthalten kann;

$X^1$ für Wasserstoff, Methyl, Ethyl, Isopropyl, Isobutyl, sek.-Butyl, tert.-Butyl, Chlor oder Fluor steht;

$X^2$ für Wasserstoff, Methyl, Ethyl, Isopropyl, Isobutyl, sek.-Butyl, tert.-Butyl, Chlor oder Fluor steht;

$X^3$ für Wasserstoff, Methyl, Ethyl, Isopropyl, Isobutyl, sek.-Butyl, tert.-Butyl, Chlor oder Fluor steht;

Y für 2-Furyl, 2-Thienyl, 2-Tetrahydrofuryl; für gegebenenfalls durch Fluor, Chlor, Methyl, Methoxy, Methylthio, Trifluormethyl, Trifluormethoxy, Trifluormethylthio, Nitro, Cyano, Phenyl und/oder Chlorphenyl substituiertes Phenyl; für 5-Methylisoxazol-3-yl, Pyrazol-1-yl, 1,2,4-Triazol-1-yl, Imidazol-1-yl; für gegebenenfalls durch Cyano substituiertes Methyl, Ethyl, Allyl oder Propargyl; für Cyclopropyl, Cyclobutyl, Cyclopentyl, Cyclohexyl, Cyclobutenyl, Cyclopentenyl, Cyclohexenyl oder Cycloheptenyl steht, die jeweils durch Methyl, Ethyl, Vinyl, Allyl, Fluor, Chlor, Chlormethyl, Trifluormethyl, Dichlorvinyl, Methoxy und Trimethylen substituiert sein können; für Dichlormethyl sowie für die Gruppierungen

$-CH_2Az$, $-CH_2OR^8$, $-CH_2OR^9$, $-CH_2SR^8$, $-CH_2SR^9$, $-CH_2SO_nR^8$, $-CH_2SO_nR^9$, $-CH_2OSO_2R^8$, $-CH_2OSO_2R^9$, $-CH_2R^9$, $-CH_2OCOR^8$, $-CH_2OCOR^9$, $-OR^8$, $-OR^9$, $-SR^8$, $-SR^9$ und $-NR^{10}R^{11}$

steht;

$R^8$ für Methyl, Ethyl, Allyl, Propyl, Propargyl, Methoxymethyl, Ethoxymethyl oder Ethoxyethyl steht;

$R^9$ für gegebenenfalls substituiertes Phenyl steht, wobei als Substituenten die bei Y bereits genannten infrage kommen;

$R^{10}$ und $R^{11}$ gleich oder verschieden sind und für Wasserstoff, Methyl, Ethyl, Isopropyl, Vinyl, Allyl, oder gegebenenfalls durch Fluor, Chlor, Methyl, Methoxy, Methylthio und/oder Trifluormethyl substituiertes Phenyl stehen;

Az und n die oben angegebene Bedeutung haben.

4. Verfahren zur Herstellung von N-(1-Alkenyl)-carbonsäureanilide der Formel (I)

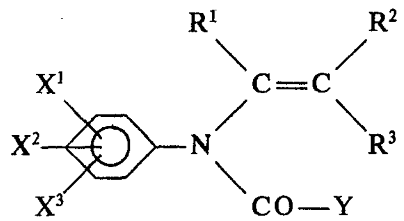

(I)

in welcher

$R^1$ für Wasserstoff und Alkyl steht,

$R^2$ für Wasserstoff, Alkyl, Alkoxy, Alkoxyalkyl, Hydroxyalkyl oder Benzyl steht,

$R^3$ für Wasserstoff, Alkyl, Alkenyl, Alkoxy, Alkoxyalkyl, Alkylcarbonyl, Alkylcarbonylalkyl, Alkoxycarbonyl oder Alkoxycarbonylalkyl steht, oder

$R^1$ und $R^2$ gemeinsam mit der C=C-Doppelbindung für einen gegebenenfalls einfach oder mehrfach durch Alkyl mit 1 bis 4 Kohlenstoffatomen; Halogen und Hydroxy substituierten, einfach oder mehrfach ungesättigten Ring stehen, der auch Heteroatome enthalten kann, oder

$R^2$ und $R^3$ gemeinsam mit dem angrenzenden Kohlenstoffatom für einen gegebenenfalls ein- oder mehrfach durch Alkyl mit 1 bis 4 Kohlenstoffatomen, Halogen und Hydroxy substituierten, gesättigten ungesättigten Ring stehen, der auch Heteroatome enthalten kann,

$X^1$, $X^2$ und $X^3$ unabhängig voneinander für Wasserstoff, Alkyl oder Halogen stehen,

Y für Furyl, Tetrahydrofuryl, Thienyl, Tetrahydrothienyl; gegebenenfalls durch Halogen, Alkyl, Alkoxy und Alkylthio mit jeweils 1 bis 4 Kohlenstoffatomen, Halogenalkyl, Halogenalkoxy und Halogenalkylthio mit jeweils 1 bis 4 Kohlenstoff- und 1 bis 5 gleichen oder verschiedenen Halogenatomen, Nitro, Cyano sowie gegebenenfalls durch Halogen und Alkyl mit 1 bis 4 Kohlenstoffatomen substituiertes Phenyl, substituiertes Phenyl; gegebenenfalls durch Alkyl substituiertes Isoxazolyl; 1,2,4-Triazol-1-yl, Imidazol-1-yl und Pyrazol-1-yl, gegebenenfalls durch Cyano oder Thiocyano substituiertes Alkyl, Alkenyl und Alkinyl; gegebenenfalls durch Alkyl, mit 1 bis 4 Kohlenstoffatomen, Alkenyl mit 2 bis 4 Kohlenstoffatomen, Halogenalkyl mit 1 bis 4 Kohlenstoff- und bis zu 5 gleichen oder verschiedenen Halogenatomen, Halogenalkenyl mit 2 bis 4 Kohlenstoff- und bis zu 5 gleichen oder verschiedenen Halogenatomen, Halogen, Alkoxy mit 1 bis 4 Kohlenstoffatomen sowie eine zwei- bis fünfgliedrige Methylenbrücke substituiertes Cycloalkyl oder Cycloalkenyl; Dihalogenalkyl; sowie die Gruppierungen

$-CH_2Az$ $-CH_2R^9$ $-CH_2OR^8$ $-CH_2OR^9$ $-CH_2SR^8$ $-CH_2SR^9$

$-CH_2-SO_nR^8$ $-CH_2SO_n-R^9$ $-CH_2OSOR^8$ $-CH_2OSOR^9$ $-CH_2OSO_2R^8$

$-CH_2OSO_2R^9$ $-CH_2OCOR^8$ $-CH_2OCOR^9$ $-OR^8$ $-OR^9$ $-SR^8$

$-SR^9$ $-NR^{10}R^{11}$ $-CH_2-O-$ 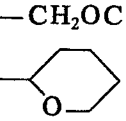

steht, wobei

$R^8$ für gegebenenfalls durch Halogen, Cyano oder Thiocyano substituiertes Alkyl, Alkenyl, Alkinyl und Alkoxyalkyl steht,

$R^9$ für gegebenenfalls durch Halogen, Alkyl, Alkoxy und Alkylthio mit jeweils 1 bis 4 Kohlenstoffatomen, Halogenalkyl, Halogenalkoxy und Halogenalkylthio mit jeweils 1 bis 4 Kohlenstoff- und 1 bis 5 gleichen oder verschiedenen Halogenatomen, Nitro, Cyano sowie gegebenenfalls durch Halogen und Alkyl mit 1 bis 4 Kohlenstoffatomen substituiertes Phenyl substituiertes Phenyl stehen,

$R^{10}$ und $R^{11}$ gleich oder verschieden sind und für Wasserstoff, Alkyl, Alkenyl oder gegebenenfalls durch Halogen, Alkyl, Alkoxy und Alkylthio mit jeweils 1 bis 4 Kohlenstoffatomen, Halogenalkyl, Halogenalkoxy und Halogenalkylthio mit jeweils 1 bis 4 Kohlenstoff- und 1 bis 5 gleichen

oder verschiedenen Halogenatomen, Nitro, Cyano sowie gegebenenfalls durch Halogen und Alkyl mit 1 bis 4 Kohlenstoffatomen substituiertes Phenyl substituiertes Phenyl stehen,

Az für Pyrazol-1-yl, 1,2,4-Triazol-1-yl und Imidazol-1-yl steht,

n für 1 oder 2 steht,

dadurch gekennzeichnet, daß man

a) Azine der Formel

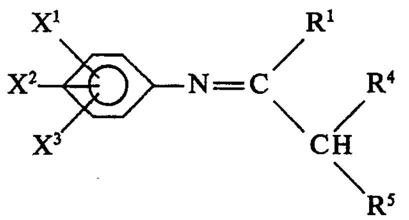

(II)

in welcher

$R^1$, $X^1$, $X^2$ und $X^3$ die oben angegebene Bedeutung haben, und

$R^4$ für Wasserstoff, Alkyl, Alkoxy, Alkoxyalkyl, Hydroxyalkyl oder Benzyl steht und

$R^5$ für Wasserstoff, Alkyl, Alkenyl, Alkoxy, Alkoxyalkyl, Alkylcarbonyl, Alkylcarbonylalkyl, Alkoxycarbonyl oder Alkoxycarbonylalkyl steht, oder

$R^1$ und $R^4$ gemeinsam mit der angrenzenden C—C-Bindung für einen gegebenenfalls ein- oder mehrfach durch Alkyl mit 1 bis 4 Kohlenstoffatomen, Halogen und Hydroxy substituierten, gesättigten oder ungesättigten Ring stehen, oder

$R^4$ und $R^5$ gemeinsam mit dem angrenzenden Kohlenstoffatom für einen gegebenenfalls ein- oder mehrfach durch Alkyl mit 1 bis 4 Kohlenstoffatomen, Halogen und Hydroxy substituierten, gesättigten oder ungesättigten Ring stehen, der auch Heteroatome enthalten kann,

mit Säure-Derivaten der Formel (III)

Hal—CO—Y (III),

in welcher

Hal für Chlor oder Brom steht, und

Y die oben angegebene Bedeutung hat,

gegebenenfalls in Gegenwart eines Verdünnungsmittels umsetzt und die entstehenden Acetanilide der Formel (IV)

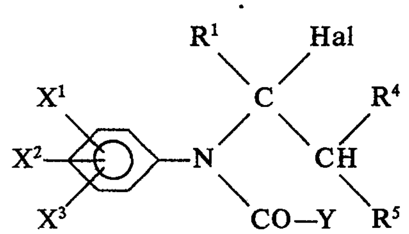

(IV)

in welcher

Hal, $R^1$, $R^4$, $R^5$, X1, $X^2$, $X^3$ und Y die oben angegebene Bedeutung haben,

ohne vorherige Isolierung, gegebenenfalls in Gegenwart einer Base, der Halogenwasserstoffeliminierung unterwirft, oder

b) Carbamidsäurechloride der Formel (V)

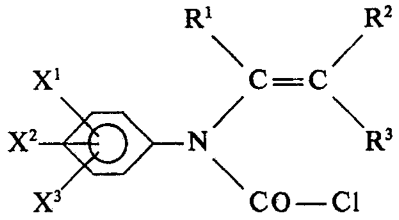

(V)

in welcher

$R^1$, $R^2$, $R^3$, $X^1$, $X^2$ und $X^3$ die oben angegebene Bedeutung haben,

mit Verbindungen der Formel (VI)

B—Y' (VI),

in welcher

Y' für Az sowie die Gruppierungen $-OR^8$, $-OR^9$, $-SR^8$, $-SR^9$ und $-NR^{10}R^{11}$ steht, wobei Az, $R^8$, $R^9$, $R^{10}$ und $R^{11}$ die oben angegebene Bedeutung haben und

B für Wasserstoff oder ein Alkalimetall steht,

gegebenenfalls in Gegenwart eines Verdünnungsmittels und gegebenenfalls in Gegenwart eines Säurebinders umsetzt, oder

c) Chloracetamide der Formel (VII)

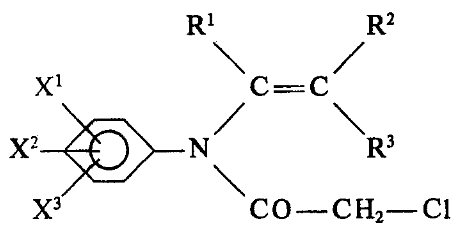

(VII)

in welcher

$R^1$, $R^2$, $R^3$, $X^1$, $X^2$ und $X^3$ die oben angegebene Bedeutung haben,

mit Verbindungen der Formel (VIII)

B—Y'' (VIII),

in welcher

Y'' für Az sowie die Gruppierungen $-OR^8$, $-OR^9$, $-SR^8$, $-SR^9$, $-OCOR^8$, $-OCOR^9$, $-OSOR^8$, $-OSOR^9$, $OSO_2R^8$, $-OSO_2R^9$ steht, wobei Az, B, $R^8$ und $R^9$ die oben angegebene Bedeutung haben,

gegebenenfalls in Gegenwart eines Verdünnungsmittels und gegebenenfalls in Gegenwart eines Säurebindemittels umsetzt, oder

d) Hydroxyacetanilide der Formel (IX)

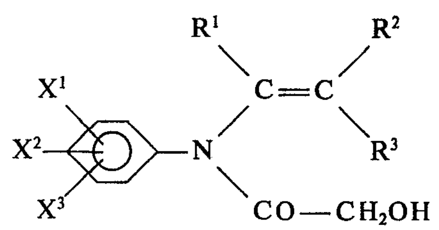

(IX)

in welcher

$R^1$, $R^2$, $R^3$, $X^1$, $X^2$ und $X^3$ die oben angegebene Bedeutung haben,

gegebenenfalls nach Aktivierung mittels Alkalimetall, mit Halogeniden der Formel (X)

$$Hal-Y''' \qquad (X),$$

in welcher

Y‴ für $R^8$ oder die Gruppierungen $-SO_2R^8$ und $-SO_2R^9$ steht, wobei
$R^8$ und $R^9$ die oben angegebene Bedeutung haben und
Hal für Chlor oder Brom steht,

in Gegenwart eines Verdünnungsmittels und gegebenenfalls in Gegenwart eines Säurebindemittels umsetzt, oder

e) erfindungsgemäß Acetanilide der Formel (XI)

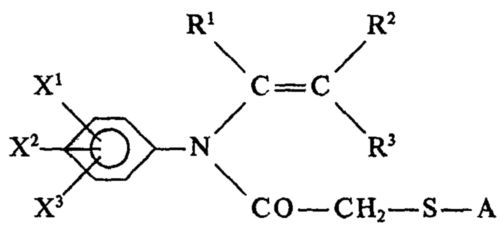

(XI)

in welcher

A für $R^8$ bzw. $R^9$ steht und
$R^1$, $R^2$, $R^3$, $R^8$, $R^9$, $X^1$, $X^2$ und $X^3$ die oben angegebene Bedeutung haben,

nach bekannten Methoden in üblicher Weise oxidiert.

5. 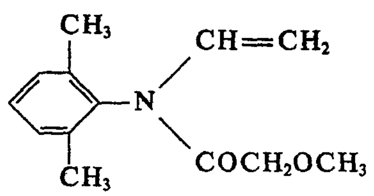

6. 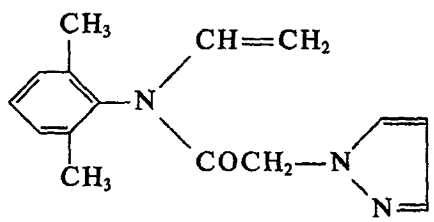

7. Fungizide Mittel, gekennzeichnet durch einen Gehalt an mindestens einem N-(Alkenyl)-carbonsäureanilid der Formel I.

8. Verfahren zur Bekämpfung von Pilzen, dadurch gekennzeichnet, daß man N-(1-Alkenyl)-carbonsäureanilid der Formel I auf Pilze oder ihren Lebensraum einwirken läßt.

9. Verwendung von N-(1-Alkenyl)-carbonsäureaniliden der Formel I zur Bekämpfung von Pilzen.

10. Verfahren zur Herstellung von fungiziden Mitteln, dadurch gekennzeichnet, daß man N-(1-Alkenyl)-carbonsäureanilid der Formel I mit Streckmitteln und/oder oberflächenaktiven Mitteln vermischt.

**Claims**

1. N-(1-Alkenyl)-carboxanilides of the general formula (I)

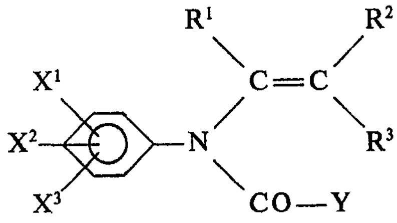

(I)

in which

$R^1$ represents hydrogen or alkyl,
$R^2$ represents hydrogen, alkyl, alkoxy, alkoxyalkyl, hydroxyalkyl or benzyl,
$R^3$ represents hydrogen, alkyl, alkenyl, alkoxy, alkoxyalkyl, alkylcarbonyl, alkylcarbonylalkyl, alkoxycarbonyl or alkoxycarbonylalkyl, or
$R^1$ and $R^2$, together with the C=C double bond, represent a mono- or polyunsaturated ring which is optionally mono- or polysubstituted by alkyl with 1 to 4 carbon atoms, halogen or hydroxyl, and which can also contain hetero atoms, or
$R^2$ and $R^3$, together with the adjacent carbon atom, represent a saturated or unsaturated ring which is optionally mono- or polysubstituted by alkyl with 1 to 4 carbon atoms, halogen or hydroxyl, and which can also contain hetero atoms,
$X^1$, $X^2$ and $X^3$ independently of one another represent hydrogen, alkyl or halogen,
Y represents furyl, tetrahydrofuryl, thienyl, tetrahydrothienyl; phenyl which is optionally substituted by halogen, alkyl, alkoxy or alkylthio with in each case 1 to 4 carbon atoms, halogenoalkyl, halogenoalkoxy or halogenoalkylthio with in each case 1 to 4 carbon and 1 to 5 identical or different halogen atoms, nitro, cyano or phenyl which is optionally substituted by halogen or alkyl with 1 to 4 carbon atoms; isoxazolyl optionally substituted by alkyl; 1,2,4-triazol-1-yl, imidazol-1-yl or pyrazol-1-yl, or alkyl, alkenyl or alkinyl which are optionally substituted by cyano or thiocyano; cycloalkyl or cycloalkenyl which are optionally substituted by alkyl with 1 to 4 carbon atoms, alkenyl with 2 to 4 carbon atoms, halogenoalkyl with 1 to 4 carbon and up to 5 identical or different halogen atoms, halogenoalkenyl with 2 to 4 carbon and up to 5 identical or different halogen atoms, halogen, alkoxy with 1 to 4 carbon atoms or a two-to five-membered methylene bridge dihalogenoalkyl; or the groupings:

$-CH_2Az$ $-CH_2R^9$ $-CH_2OR^8$ $-CH_2OR^9$ $-CH_2SR^8$ $-CH_2SR^9$

$-CH_2-SO_nR^8$ $-CH_2SO_n-R^9$ $-CH_2OSOR^8$ $-CH_2OSOR^9$ $-CH_2OSO_2R^8$

$-CH_2OSO_2R^9$ $-CH_2OCOR^8$ $-CH_2OCOR^9$ $-OR^8$ $-OR^9$ $-SR^8$

$-SR^9$ $-NR^{10}R^{11}$ or $-CH_2-O-$ 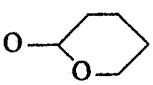

wherein
$R^8$ represents optionally halogen-, cyano- or thiocyanosubstituted alkyl, alkenyl, alkinyl or alkoxyalkyl,
$R^9$ represents phenyl which is optionally substituted by halogen, alkyl, alkoxy or alkylthio with in each case 1 to 4 carbon atoms, halogenoalkyl, halogenoalkoxy or halogenoalkylthio with in each case 1 to 4 carbon and 1 to 5 identical or different halogen atoms, nitro, cyano, or phenyl which is optionally substituted by halogen or alkyl with 1 to 4 carbon atoms,
$R^{10}$ and $R^{11}$ are identical or different and represent hydrogen, alkyl, alkenyl or phenyl which is optionally substituted by halogen, alkyl, alkoxy or alkylthio with in each case 1 to 4 carbon atoms, halogenoalkyl, halogenoalkoxy or halogenoalkylthio with in each case 1 to 4 carbon and 1 to 5 identical or different halogen atoms, nitro, cyano, or phenyl which is optionally substituted by halogen or alkyl with 1 to 4 carbon atoms,
Az represents pyrazol-1-yl, 1,2,4-triazol-1-yl or imidazol-1-yl-
n represents 1 or 2,

and, when

Y represents Az or $-CH_2-Az$, the physiologically tolerated acid addition salts and metal salt complexes thereof.

2. N-(1-Alkenyl)-carboxanilides of the formula (I), in which

$R^1$ represents hydrogen or straight-chain or branched alkyl with 1 to 4 carbon atoms;

$R^2$ represents hydrogen, straight-chain or branched alkyl with 1 to 4 carbon atoms, alkoxy with 1 to 4 carbon atoms, hydroxyalkyl with 1 to 4 carbon atoms, alkoxyalkyl with 1 to 4 carbon atoms in the alkyl part and 1 to 4 carbon atoms in the alkoxy part or benzyl;

$R^3$ represents hydrogen, straight-chain or branched alkyl with 1 to 4 carbon atoms, alkoxy with 1 to 4 carbon atoms, alkoxyalkyl with 1 to 4 carbon atoms in the alkyl part and 1 to 4 carbon atoms in the alkoxy part, alkylcarbonyl with 1 to 4 carbon atoms in the alkyl part, alkylcarbonylalkyl with 1 to 4 carbon atoms in each alkyl part, alkoxycarbonyl with 1 to 4 carbon atoms in the alkoxy part, alkoxycarbonylalkyl with 1 to 4 carbon atoms in the alkoxy part and 1 to 4 carbon atoms in the alkyl part or straight-chain or branched alkenyl with 2 to 6 carbon atoms;

or

$R^1$ and $R^2$, together with the C=C double bond, represent an optionally mono- or polysubstituted, mono- or polyunsaturated, 5- or 6-membered ring which can contain one or two hetero atoms, the substituents which may be mentioned being: alkyl with 1 to 4 carbon atoms, halogen or hydroxyl;

or

$R^2$ and $R^3$, together with the adjacent carbon atom, represent an optionally mono- or polysubstituted, saturated or unsaturated, 5- or 6-membered ring which can contain one or two hetero atoms, the substituents which may be mentioned being: alkyl with 1 to 4 carbon atoms, halogen or hydroxyl;

$X^1$ represents hydrogen, straight-chain or branched alkyl with 1 to 4 carbon atoms, fluorine, chlorine or bromine;

$X^2$ represents hydrogen, straight-chain or branched alkyl with 1 to 4 carbon atoms, fluorine, chlorine or bromine; and

$X^3$ represents hydrogen or straight-chain alkyl with 1 to 4 carbon atoms, fluorine, chlorine or bromine,

Y represents furyl, tetrahydrofuryl, thienyl, tetrahydrothienyl, optionally substituted phenyl, the substituents which may be mentioned being: halogen, alkyl, alkoxy or alkylthio with in each case 1 to 4 carbon atoms, halogenoalkyl, halogenalkoxy or halogenoalkylthio with in each case 1 to 4 carbon and 1 to 5 identical or different fluorine or chlorine atoms, nitro, cyano, or phenyl which is optionally substituted by halogen or alkyl with 1 to 4 carbon atoms, isoxazolyl which is optionally substituted by methyl or ethyl, 1,2,4-triazol-1-yl, imidazol-1-yl or pyrazol-1-yl, optionally cyano- or thiocyano-substituted alkyl with 1 to 4 carbon atoms or alkenyl or alkinyl with in each case 2 to 4 carbon atoms, optionally substituted cycloalkyl with 3 to 7 carbon atoms or cycloalkenyl with 4 to 7 carbon atoms, possible substituents being: alkyl with 1 to 4 carbon atoms, alkenyl with 2 to 4 carbon atoms, halogenoalkyl with 1 to 4 carbon and up to 5 identical or different halogen atoms, halogenoalkenyl with 2 to 4 carbon and up to 5 identical or different halogen atoms, halogen, alkoxy with 1 to 4 carbon atoms or a two- to fivemembered methylene bridge; furthermore dihalogenoalkyl with 1 to 2 carbon atoms, the halogen atoms which may be mentioned being fluorine, chlorine or bromine; or the groupings

$-CH_2Az$ $-CH_2R^9$ $-CH_2OR^8$ $-CH_2OR^9$ $-CH_2SR^8$ $-CH_2SR^9$

$-CH_2-SO_nR^8$ $-CH_2SO_nR^9$ $-CH_2OSOR^8$ $-CH_2OSOR^9$ $-CH_2OSO_2R^8$

$-CH_2OSO_2R^9$ $-CH_2OCOR^8$ $-CH_2OCOR^9$ $-OR^8$ $-OR^9$ $-SR^8$

$-SR^9$ $-NR^{10}R^{11}$ or $-CH_2-O-$ 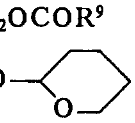

$R^8$ represents optionally fluorine-, chlorine-, or bromine-, or cyano- or thiocyano-substituted alkyl with 1 to 4 or alkenyl or alkinyl with in each case 2 to 4 carbon atoms or alkoxyalkyl with 1 to 4 carbon atoms in each alkyl part;

$R^9$ represents optionally substituted phenyl, possible phenyl substituents being those already mentioned under Y;

$R^{10}$ and $R^{11}$ represent hydrogen, alkyl with 1 to 4 carbon atoms, alkenyl with 2 to 4 carbon atoms or optionally substituted phenyl, possible phenyl substituents being those already mentioned under Y;

Az represents pyrazol-1-yl, 1,2,4-triazol-1-yl or imidazol-1-yl; and the index

n represents 1 or 2.

3. N-(1-Alkenyl)-carboxyanilides of the formula (I), in which

$R^1$ represents hydrogen, methyl, ethyl or isopropyl;
$R^2$ represents hydrogen, methyl, ethyl, isopropyl, methoxy, ethoxy, isopropoxy, methoxymethyl, ethoxymethyl, isohydroxypropyl or benzyl;
$R^3$ represents hydrogen, methyl, ethyl, isopropyl, methoxy, ethoxy, isopropoxy, methoxymethyl, ethoxymethyl, methylcarbonyl, ethylcarbonyl, methylcarbonylmethyl, methoxycarbonyl, ethoxycarbonyl, methoxycarbonylmethyl or straight-chain or branched alkenyl with 2 to 4 carbon atoms;
or
$R^1$ and $R^2$, together with the C=C double bond, represent a mono- or polyunsaturated 5- or 6-membered ring which is optionally substituted by methyl, ethyl, hydroxyl and/or chlorine and which can contain one or two oxygen, nitrogen and/or sulphur atoms;
or
$R^2$ and $R^3$, together with the adjacent carbon atoms, represent a saturated or unsaturated 5- or 6-membered ring which is optionally substituted by methyl, ethyl, hydroxyl and/or chlorine and which can contain one or two oxygen, nitrogen or sulphur atoms;
$X^1$ represents hydrogen, methyl, ethyl, isopropyl, isobutyl, sec.-butyl, tert.-butyl, chlorine or fluorine;
$X^2$ represents hydrogen, methyl, ethyl, isopropyl, isobutyl, sec.-butyl, tert.-butyl, chlorine or fluorine;
$X^3$ represents hydrogen, methyl, ethyl, isopropyl, isobutyl, sec.-butyl, tert.-butyl, chlorine or fluorine;
Y represents 2-furyl, 2-thienyl or 2-tetrahydrofuryl; phenyl which is optionally substituted by fluorine, chlorine, methyl, methoxy, methylthio, trifluoromethyl, trifluoromethoxy, trifluoromethylthio, nitro, cyano, phenyl and/or chlorophenyl; 5-methyl-isoxazol-3-yl, pyrazol-1-yl, 1,2,4-triazol-1-yl or imidazol-1-yl; optionally cyano-substituted methyl, ethyl, allyl or propargyl; cyclopropyl, cyclobutyl, cyclopentyl, cyclohexyl, cyclobutenyl, cyclopentenyl, cyclohexenyl or cycloheptenyl, which can each be substituted by methyl, ethyl, vinyl, allyl, fluorine, chlorine, chloromethyl, trifluoromethyl, dichlorovinyl, methoxy or trimethylene; or dichloromethyl or the groupings $-CH_2Az$, $-CH_2OR^8$, $-CH_2OR^9$, $-CH_2SR^8$, $-CH_2SR^9$, $-CH_2SO_nR^8$, $-CH_2SO_nR^9$, $-CH_2OSO_2R^8$, $-CH_2OSO_2R^9$, $-CH_2R^9$, $-CH_2OCOR^9$, $-CH_2OCOR^9$, $-OR^8$, $-OR^9$, $-SR^8$, $-SR^9$ or $-NR^{10}R^{11}$;
$R^8$ represents methyl, ethyl, allyl, propyl, propargyl, methoxymethyl, ethoxymethyl or ethoxyethyl;
$R^9$ represents optionally substituted phenyl, possible substituents being those already mentioned under Y;
$R^{10}$ and $R^{11}$ are identical or different and represent hydrogen, methyl, ethyl, isopropyl, vinyl, allyl, or phenyl which is optionally substituted by fluorine, chlorine, methyl, methoxy, methylthio and/or trifluoromethyl; and
Az and n have the abovementioned meaning.

4. Process for the preparation of N-(1-alkenyl)-carboxanilides of the formula (I)

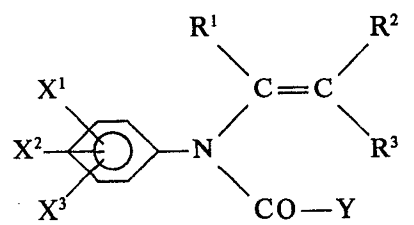

(I)

in which

$R^1$ represents hydrogen or alkyl,
$R^2$ represents hydrogen, alkyl, alkoxy, alkoxyalkyl, hydroxyalkyl or benzyl,
$R^3$ represents hydrogen, alkyl, alkenyl, alkoxy, alkoxyalkyl, alkylcarbonyl, alkylcarbonylalkyl, alkoxycarbonyl or alkoxycarbonylalkyl, or
$R^1$ and $R^2$, together with the C=C double bond, represent a mono- or polyunsaturated ring which is optionally mono- or polysubstituted by alkyl with 1 to 4 carbon atoms, halogen or hydroxyl, and which can also contain hetero atoms, or
$R^2$ and $R^3$, together with the adjacent carbon atom represent a saturated or unsaturated ring which is optionally mono- or polysubstituted by alkyl with 1 to 4 carbon atoms, halogen or hydroxyl, and which can also contain hetero atoms,
$X^1$, $X^2$ and $X^3$ independently of one another represent hydrogen, alkyl or halogen,
Y represents furyl, tetrahydrofuryl, thienyl, tetrahydrothienyl; phenyl which is optionally substituted by halogen, alkyl, alkoxy or alkylthio with in each case 1 to 4 carbon atoms, halogenoalkyl, halogenoalkoxy or halogenalkylthio with in each case 1 to 4 carbon and 1 to 5 identical or different

halogen atoms, nitro, cyano or phenyl which is optionally substituted by halogen or alkyl with 1 to 4 carbon atoms; isoxazolyl optionally substituted by alkyl; 1,2,4-triazol-1-yl, imidazol-1-yl or pyrazol-1-yl, or alkyl, alkenyl or alkinyl which are optionally substituted by cyano or thiocyano; cycloalkyl or cycloalkenyl which are optionally substituted by alkyl with 1 to 4 carbon atoms, alkenyl with 2 to 4 carbon atoms, halogenoalkyl with 1 to 4 carbon and up to 5 identical or different halogen atoms, halogenoalkenyl with 2 to 4 carbon and up to 5 identical or different halogen atoms, halogen, alkoxy with 1 to 4 carbon atoms or a two-to five-membered methylene bridge; dihalogenoalkyl; or the groupings:

$-CH_2Az$ $-CH_2R^9$ $-CH_2OR^8$ $-CH_2OR^9$ $-CH_2SR^8$ $-CH_2SR^9$

$-CH_2-SO_nR^8$ $-CH_2SO_n-R^9$ $-CH_2OSOR^8$ $-CH_2OSOR^9$ $-CH_2OSO_2R^8$

$-CH_2OSO_2R^9$ $-CH_2OCOR^8$ $-CH_2OCOR^9$ $-OR^8$ $-OR^9$ $-SR^8$

$-SR^9$ $-NR^{10}R^{11}$ or 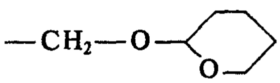

wherein

$R^8$ represents optionally halogen-, cyano- or thiocyanosubstituted alkyl, alkenyl, alkinyl or alkoxyalkyl,

$R^9$ represents phenyl which is optionally substituted by halogen, alkyl, alkoxy or alkylthio with in each case 1 to 4 carbon atoms, halogenoalkyl, halogenoalkoxy or halogenoalkylthio with in each case 1 to 4 carbon and 1 to 5 identical or different halogen atoms, nitro, cyano, or phenyl which is optionally substituted by halogen or alkyl with 1 to 4 carbon atoms,

$R^{10}$ and $R^{11}$ are identical or different and represent hydrogen, alkyl, alkenyl or phenyl which is optionally substituted by halogen, alkyl, alkoxy or alkylthio with in each case 1 to 4 carbon atoms, halogenoalkyl, halogenoalkoxy or halogenoalkylthio with in each case 1 to 4 carbon and 1 to 5 identical or different halogen atoms, nitro, cyano, or phenyl which is optionally substituted by halogen or alkyl with 1 to 4 carbon atoms,

Az represents pyrazol-1-yl, 1,2,4-triazol-1-yl or imidazol-1-yl, and

n represents 1 or 2,

characterised in that

a) azines of the formula

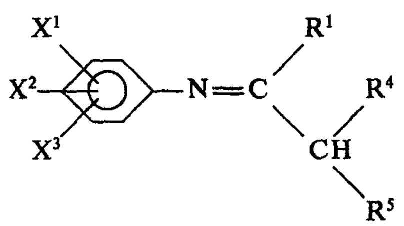

(II)

in which

$R^1$, $X^1$, $X^2$ and $X^3$ have the abovementioned meaning, and

$R^4$ represents hydrogen, alkyl, alkoxy, alkoxyalkyl, hydroxyalkyl or benzyl and

$R^5$ represents hydrogen, alkyl, alkenyl, alkoxy, alkoxyalkyl, alkylcarbonyl, alkylcarbonylalkyl, alkoxycarbonyl or alkoxycarbonylalkyl, or

$R^1$ and $R^4$, together with the adjacent C—C bond, represent a saturated or unsaturated ring which is optionally mono or polysubstituted by alkyl with 1 to 4 carbon atoms, halogen or hydroxyl, or

$R^4$ and $R^5$, together with the adjacent carbon atom, represent a saturated or unsaturated ring which is optionally mono- or polysubstituted by alkyl with 1 to 4 carbon atoms, halogen or hydroxyl and which can also contain hetero atoms,

are reacted with acid derivatives of the formula (III)

Hal—CO—Y (III),

in which

Hal represents chlorine or bromine, and
Y has the abovementioned meaning,

if appropriate in the presence of a diluent, and the resulting acetanilides of the formula (IV)

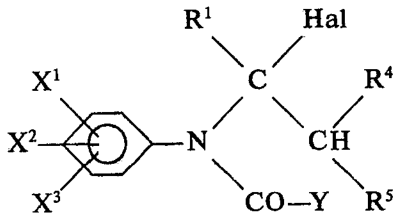

(IV)

in which

Hal, $R^1$, $R^4$, $R^5$, $X^1$, $X^2$, $X^3$ and Y have the abovementioned meaning,

are subjected, without prior isolation, to hydrogen halide elimination, if appropriate in the presence of a base, or

b) carbamic acid chlorides of the formula (V)

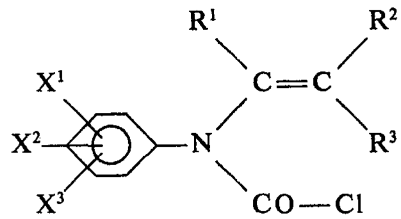

(V)

in which

$R^1$, $R^2$, $R^3$, $X^1$, $X^2$ and $X^3$ have the abovementioned meaning,

are reacted with compounds of the formula (VI)

B—Y' (VI),

in which

Y' represents Az or the groupings $-OR^8$, $-OR^9$, $-SR^8$, $-SR^9$ or $-NR^{10}R^{11}$, wherein Az, $R^8$, $R^9$, $R^{10}$ and $R^{11}$ have the abovementioned meaning and
B represents hydrogen or an alkali metal,

if appropriate in the presence of a diluent and if appropriate in the presence of an acid-binder, or

c) chloroacetamides of the formula (VII)

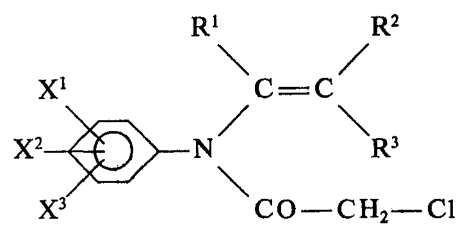

(VII)

in which

$R^1$, $R^2$, $R^3$, $X^1$, $X^2$ and $X^3$ have the abovementioned meaning,

are reacted with compounds of the formula (VIII)

B—Y'' (VIII),

in which

Y'' represents Az or the groupings $-OR^8$, $-OR^9$, $-SR^8$, $-SR^9$, $-OCOR^8$, $-OCOR^9$, $-OSOR^8$, $-OSO_2R^8$, or $-OSO_2R^9$,
wherein
Az, B, $R^8$ and $R^9$ have the abovementioned meaning,

if appropriate in the presence of a diluent and if appropriate in the presence of an acid-binding agent, or

d) hydroxyacetanilides of the formula (IX)

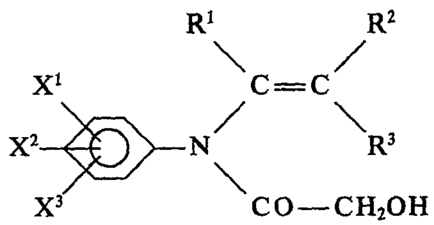

(IX)

in which

$R^1$, $R^2$, $R^3$, $X^1$, $X^2$ and $X^3$ have the abovementioned meaning,

are reacted, if appropriate after being activated by means of an alkali metal, with halides of the formula (X)

$$Hal-Y''' \quad (X),$$

in which

Y''' represents $R^8$ or the groupings $-SO_2R^8$ or $-SO_2R^9$, wherein
$R^8$ and $R^9$ have the abovementioned meaning and
Hal represents chlorine or bromine,

in the presence of a diluent and if appropriate in the presence of an acid-binding agent, or

e) acetanilides, according to the invention, of the formula (XI)

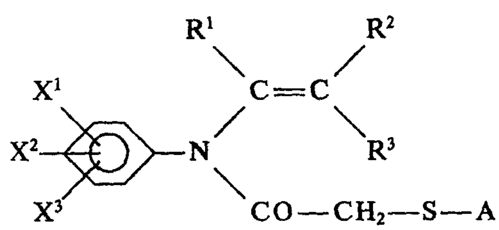

(XI)

in which

A represents $R^8$ or $R^9$ and
$R^1$, $R^2$, $R^3$, $R^8$, $R^9$, $X^1$, $X^2$ and $X^3$ have the abovementioned meaning,

are oxidised in a customary manner by known methods.

5. 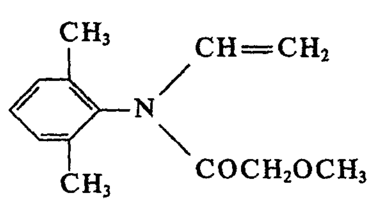

6. 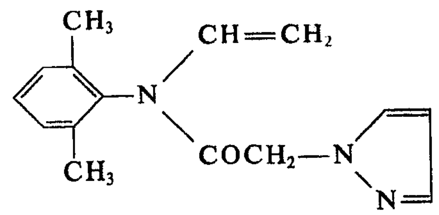

7. Fungicidal agents, characterised in that they contain at least one N-(alkenyl)-carboxanilide of the formula I.

8. Method of combating fungi, characterised in that N-(1-alkenyl)-carboxanilide of the formula I is allowed to act on fungi or their environment.

9. Use of N-(1-alkenyl)-carboxanilides of the formula I for combating fungi.

10. Process for the preparation of fungicidal agents, characterised in that N-(1-alkenyl)-carboxanilide of the formula I is mixed with extenders and/or surface-active agents.

**Revendications**

1. N-(1-Alcényl)-anilides d'acides carboxyliques de formule générale (I)

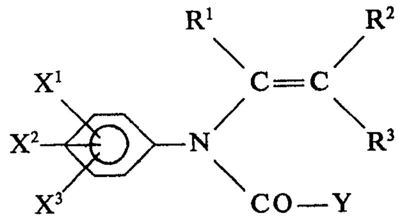

(I)

dans laquelle

$R^1$ représente un atome d'hydrogène et un groupe alkyle,

$R^2$ représente un atome d'hydrogène, un groupe alkyle, alcoxy, alcoxyalkyle, hydroxyalkyle ou benzyle,

$R^3$ représente un atome d'hydrogène, un groupe alkyle, alcényle, alcoxy, alcoxyalkyle, alkylcarbonyle, alkylcarbonylalkyle, alcoxycarbonyle ou alcoxycarbonylalkyle, ou

$R^1$ et $R^2$ pris avec la double liaison C=C représente un noyau à une ou plusieurs insaturations, substitué éventuellement une ou plusieurs fois par un groupe alkyle ayant 1 à 4 atomes de carbone, par de l'halogène et un groupe hydroxyle, et qui peut contenir aussi des hétéroatomes, ou

$R^2$ et $R^3$, pris avec l'atome de carbone qui les réunit, peuvent représenter un noyau saturé ou insaturé, éventuellement substitué une ou plusieurs fois par un groupe alkyle ayant 1 à 4 atomes de carbone, par de l'halogène et un groupe hydroxyle, et qui peut aussi contenir des hétéroatomes,

$X^1$, $X^2$ et $X^3$ représentent chacun, indépendamment l'un de l'autre, un atome d'hydrogène, un groupe alkyle ou à atome d'halogène,

Y représente un groupe furyle, tétrahydrofuryle, thiényle, tétrahydrothiényle; un noyau phényle éventuellement substitué par de l'halogène, par un groupe alkyle, alcoxy et alkylthio ayant chacun 1 à 4 atomes de carbone, halogénoalkyle, halogénoalcoxy et halogénoalkylthio ayant chaque fois 1 à 4 atomes de carbone et 1 à 5 atomes d'halogène identiques ou différents, nitro, cyano, ainsi que par un groupe phényle éventuellement substitué par de l'halogène et par un groupe alkyle ayant 1 à 4 atomes de carbone; un groupe isoxazolyle éventuellement substitué par un groupe alkyle; 1,2,4-triazol-1-yle, imidazol-1-yle et pyrazol-1-yle, alkyle, alcényle et alcinyle éventuellement substitué par un groupe cyano ou thiocyano; cycloalkyle ou cycloalcényle éventuellement substitué par un groupe alkyle ayant 1 à 4 atomes de carbone, alcényle ayant 2 à 4 atomes de carbone, halogénoalkyle ayant 1 à 4 atomes de carbone et jusqu'à 5 atomes d'halogène identiques ou différents, halogénoalcényle ayant 2 à 4 atomes de carbone et jusqu'à 5 atomes d'halogène identiques ou différents, par de l'halogène, par un groupe alcoxy ayant 1 à 4 atomes de carbone ainsi que par un pont méthylénique comportant 2 à 5 chaînons; un groupe dihalogénoalkyle; ainsi que les groupements

$-CH_2Az$   $-CH_2R^9$   $-CH_2OR^8$   $-CH_2OR^9$   $-CH_2SR^8$   $-CH_2SR^9$

$-CH_2-SO_nR^8$   $-CH_2SO_n-R^9$   $-CH_2OSOR^8$   $-CH_2OSOR^9$   $-CH_2OSO_2R^8$

$-CH_2OSO_2R^9$   $-CH_2OCOR^8$   $-CH_2OCOR^9$   $-OR^8$   $-OR^9$   $-SR^8$

$-SR^9$   $-NR^{10}R^{11}$   $-CH_2-O-$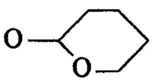

où

$R^8$ représente un groupe alkyle, alcényle, alcynyle ou alcoxyalkyle éventuellement substitués par de l'halogène, par un groupe cyano ou thiocyano;

$R^9$ représente un groupe phényle éventuellement substitué par de l'halogène, par un groupe alkyle, alcoxy et alkylthio ayant chacun 1 à 4 atomes de carbone, halogénoalkyle, halogénoalcoxy et halogénoalkylthio ayant chacun 1 à 4 atomes de carbone et 1 à 5 atomes d'halogène identiques ou différents, nitro, cyano ainsi que par un noyau phényle éventuellement substitué par de l'halogène et par un groupe alkyle ayant 1 à 4 atomes de carbone;

$R^{10}$ et $R^{11}$ sont identiques ou différents et représentent chacun un atome d'hydrogène, un groupe alkyle, alcényle ou phényle (éventuellement substitué par de l'halogène, par un groupe alkyle, alcoxy et alkylthio ayant chacun 1 à 4 atomes de carbone, halogénoalkyle, halogénoalcoxy et halogénoalkylthio ayant chacun 1 à 4 atomes de carbone et 1 à 5 atomes d'halogène identiques ou différents), un groupe nitro, cyano, ainsi qu'un noyau phényle (éventuellement substitué par de l'halogène ou par un groupe alkyle ayant 1 à 4 atomes de carbone),

Az représente un groupe pyrazol-1-yle, 1,2,4-triazol-1-yle et imidazol-1-yle,

n vaut 1 ou 2,

et, lorsque

Y représente Az ou $-CH_2-Az$, leurs sels d'addition d'acide et leurs complexes avec des sels de métaux, physiologiquement tolérables.

2. N-(1-Alcényl)-anilides d'acides carboxyliques de formule (I), dans laquelle

$R^1$ représente un atome d'hydrogène ou un groupe alkyle linéaire ou ramifié ayant 1 à 4 atomes de carbone;

$R^2$ représente un atome d'hydrogène, un groupe alkyle linéaire ou ramifié ayant 1 à 4 atomes de carbone, alcoxy ayant 1 à 4 atomes de carbone, hydroxyalkyle ayant 1 à 4 atomes des carbone, alcoxyalkyle ayant 1 à 4 atomes de carbone dans la partie alkyle et 1 à 4 atomes de carbone dans la partie alcoxy, ou un groupe benzyle;

$R^3$ représente un atome d'hydrogène, un groupe alkyle linéaire ou ramifié ayant 1 à 4 atomes de carbone, alcoxy ayant 1 à 4 atomes de carbone, alcoxyalkyle ayant 1 à 4 atomes de carbone dans la partie alkyle et 1 à 4 atomes de carbone dans la partie alcoxy, alkylcarbonyle ayant 1 à 4 atomes de carbone dans la partie alkyle, alkylcarbonylalkyle ayant 1 à 4 atomes de carbone dans chaque partie alkyle, alcoxycarbonyle ayant 1 à 4 atomes de carbone dans la partie alcoxy, alcoxycarbonylalkyle ayant 1 à 4 atomes de carbone dans la partie alcoxy et 1 à 4 atomes de carbone dans la partie alkyle, ou un groupe alcényle linéaire ramifié ayant 2 à 6 atomes de carbone;

ou bien

$R^1$ et $R^2$ pris avec la double liaison $C=C$, représente un noyau à 5 ou 6 chaînons, éventuellement substitué une ou plusieurs fois, insaturé une ou plusieurs fois, qui peut contenir 1 ou 2 hétéroatomes, et l'on peut citer comme substituant: un groupe alkyle ayant 1 à 4 atomes de carbone, de l'halogène et un groupe hydroxyle;

ou bien

$R^2$ et $R^3$ pris avec l'atome de carbone qui les réunit, peuvent former un noyau à 5 ou 6 chaînons, saturé ou insaturé, éventuellement substitué une ou plusieurs fois, qui peut contenir 1 ou 2 hétéroatomes, et l'on peut citer comme substituant un groupe alkyle ayant 1 à 4 atomes de carbone, de l'halogène et un groupe hydroxyle;

$X^1$ représente un atome d'hydrogène, un groupe alkyle linéaire ou ramifié ayant 1 à 4 atomes de carbone, du fluor, du chlore ou du brome;

$X^2$ représente un atome d'hydrogène, un groupe alkyle linéaire ou ramifié ayant 1 à 4 atomes de carbone, du fluor, du chlore ou du brome; et

$X^3$ représente un atome d'hydrogène et un groupe alkyle linéaire ayant 1 à 4 atomes de carbone, du fluor, du chlore ou du brome,

Y représente un groupe furyle, tétrahydrofuryle, thiényle, tétrahydrothiényle, phényle éventuellement substitué, et l'on peut alors citer comme substituant: un halogène, un groupe alkyle, alcoxy et alkylthio ayant chacun 1 à 4 atomes de carbone, halogénoalkyle, halogénoalcoxy et halogénoalkylthio ayant chacun 1 à 4 atomes de carbone et 1 à 5 atomes de fluor ou de chlore, identiques ou différents, un groupe nitro, cyano ainsi qu'un groupe phényle (éventuellement substitué par de l'halogène et par un groupe alkyle ayant 1 à 4 atomes de carbone), Y peut représenter un groupe isoxazolyle éventuellement substitué par un groupe méthyle ou éthyle, un groupe 1,2,4-triazol-1-yle, imidazol-1-yle et pyrazol-1-yle, un groupe alkyle ayant 1 à 4 atomes de carbone et éventuellement substitué par un groupe cyano ou thiocyano, ainsi qu'un groupe alcényle et alcynyle ayant chacun 2 à 4 atomes de carbone; un groupe cycloalkyle ayant 3 à 7 atomes de carbone ou cycloalcényle ayant 4 à 7 atomes de carbone, éventuellement substitués et on peut citer comme substituant: un groupe alkyle ayant 1 à 4 atomes de carbone, alcényle ayant 2 à 4 atomes de carbone, halogénoalkyle ayant 1 à 4 atomes de carbone et jusqu'à 5 atomes d'halogène identiques ou différents, halogénoalcényle ayant 2 à 4 atomes de carbone et jusqu'à 5 atomes d'halogène identiques ou différents, de l'halogène, un groupe alcoxy ayant 1 à 4 atomes de carbone ainsi qu'un pont méthylénique comportant 2 à 5 chaînons; Y peut représenter en outre un groupe dihalogénoalkyle ayant 1 à 2 atomes de carbone, et l'on peut citer comme atome d'halogène le fluor, le chlore et la brome; ainsi que les groupements

$-CH_2Az$ $-CH_2R^9$ $-CH_2OR^8$ $-CH_2OR^9$ $-CH_2SR^8$ $-CH_2SR^9$

$-CH_2-SO_nR^8$ $-CH_2SO_nR^9$ $-CH_2OSOR^8$ $-CH_2OSOR^9$ $-CH_2OSO_2R^8$

$-CH_2OSO_2R^9$ $-CH_2OCOR^8$ $-CH_2OCOR^9$ $-OR^8$ $-OR^9$ $-SR^8$

$-SR^9$ $-NR^{10}R^{11}$ et $-CH_2-O-$ 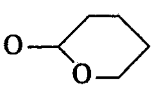

$R^8$ représente un groupe alkyle ayant 1 à 4 atomes de carbone et alcényle ou alcinyle ayant chacun 2 à 4 atomes de carbone, éventuellement substitués par du fluor, du chlore ou du brome, par un groupe cyano ou thiocyano; et un groupe alcoxyalkyle ayant 1 à 4 atomes de carbone dans chaque partie alkyle;

$R^9$ représente un noyau phényle éventuellement substitué, et l'on peut citer comme substituant ceux indiqués déjà à propos de Y;

$R^{10}$ et $R^{11}$ représentent de l'hydrogène, un groupe alkyle ayant 1 à 4 atomes de carbone, alcényle ayant 2 à 4 atomes de carbone, ainsi qu'un noyau phényle éventuellement substitué, et l'on peut citer comme substituant ceux déjà indiqués à propos de Y;

Az représente un groupe pyrazol-1-yle, 1,2,4-triazol-1-yle et imidazol-1-yle; et l'indice

n vaut 1 ou 2.

3. N-(1-Alcényl)-anilides d'acides carboxyliques de formule (I), dans laquelle

$R^1$ représente un atome d'hydrogène, un groupe méthyle, éthyle ou isopropyle;

$R^2$ représente un atome d'hydrogène, un groupe méthyle, éthyle, isopropyle, méthoxy, éthoxy, isopropoxy, méthoxyméthyle, éthoxyméthyle, isohydroxypropyle ou benzyle;

$R^3$ représente un atome d'hydrogène, un groupe méthyle, éthyle, isopropyle, méthoxy, éthoxy, isopropoxy, méthoxyméthyle, éthoxyméthyle, méthylcarbonyle, éthylcarbonyle, méthylcarbonylméthyle, méthoxycarbonyle, éthoxycarbonyle, méthoxycarbonylméthyle ainsi qu'un groupe alcényle, linéaire ou ramifié, comportant 2 à 4 atomes de carbone;

ou bien

$R^1$ et $R^2$, pris avec la double liaison C=C, représentent un noyau à 5 ou 6 chaînons, éventuellement substitué par un groupe méthyle, éthyle, hydroxyle et/ou par du chlore et il présente une ou plusieurs insaturationset peut contenir 1 ou 2 atomes d'oxygène, d'azote et/ou de soufre;

ou bien

$R^2$ et $R^3$, pris avec l'atome de carbone, qui les réunit, peuvent représenter un noyau à 5 ou 6 chaînons, saturé ou insaturé, éventuellement substitué par un groupe méthyle, éthyle, hydroxyle et/ou par du chlore et qui peut contenir 1 ou 2 atomes d'oxygène, d'azote ou de soufre;

$X^1$ représente un atome d'hydrogène, un groupe méthyle, éthyle, isopropyl, isobutyle, butyle secondaire, tertiobutyle, un atome de chlore ou de fluor;

$X^2$ représente un atome d'hydrogène, un groupe méthyle, éthyle, isopropyle, isobutyle, butyle secondaire, tertiobutyle, un atome de chlore ou de fluor;

$X^3$ représente un atome d'hydrogène, un groupe méthyle, éthyle, isopropyle, isobutyle, butyle secondaire, tertiobutyle, un atome de chlore ou de fluor;

Y représente un groupe 2-furyle, 2-thiényle, 2-tétrahydrofuryle; un noyau phényle (éventuellement substitué par du fluor, du chlore, un groupe méthyle, méthoxy, méthylthio, trifluorométhyle, trifluorométhoxy, trifluorométhylthio, nitro, cyano, phényle et/ou chlorophényle); un groupe 5-méthyl-isoxazol-3-yle, pyrazol-1-yle, 1,2,4-triazol-1-yle, imidazol-1-yle; un groupe méthyle, éthyle, allyle ou propargyle éventuellement substitués par un groupe cyano; un groupe cyclopro-

pyle, cyclobutyle, cyclopentyle, cyclohexyle, cyclobuténylе, cyclopenténylе, cyclohexényle, ou cycloheptényle, qui peuvent chacun etre substitué par un groupe méthyle, éthyle, vinyle, allyle, fluoro, chloro, chlorométhyle, trifluorométhyle, dichlorovinyle, méthoxy et triméthylène; un groupe dichlorométhyle, ainsi que les groupements
$-CH_2Az$, $-CH_2OR^8$, $-CH_2OR^9$, $-CH_2SR^8$, $-CH_2SR^9$, $-CH_2SO_nR^8$, $-CH_2SO_nR^9$, $-CH_2OSO_2R^8$, $-CH_2OSO_2R^9$, $-CH_2R^9$, $-CH_2OCOR^8$, $-CH_2OCOR^9$, $-OR^8$, $-OR^9$, $-SR^8$, $-SR^9$ et $-NR^{10}R^{11}$;

$R^8$ représente un groupe méthyle, éthyle, allyle, propyle, propargyle, méthoxyméthyle, éthoxyméthyle ou éthoxyéthyle;

$R^9$ représente un groupe phényle éventuellement substitué, et l'on peut citer comme substituant ceux déjà indiqués à propos de Y;

$R^{10}$ et $R^{11}$ sont identiques ou différents et représentent chacun un atome d'hydrogène, un groupe méthyle, éthyle, isopropyle, vinyle, allyle ou un noyau phényle (éventuellement substitué par du fluor, du chlore, un groupe méthyle, méthoxy, méthylthio et/ou trifluorométhyle);

Az et n ont le sens indiqué ci-dessus.

4. Procédé de préparation de N-(1-alcényl)-anilides d'acides carboxyliques de formule (I)

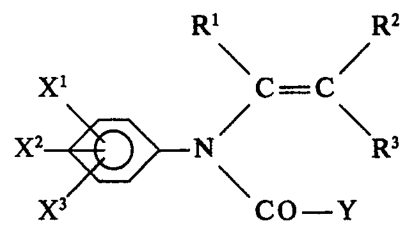

(I)

dans laquelle

$R^1$ représente un atome d'hydrogène et un groupe alkyle,

$R^2$ représente un atome d'hydrogène, un groupe alkyle, alcoxy, alcoxyalkyle, hydroxyalkyle ou benzyle,

$R^3$ représente un atome d'hydrogène, un groupe alkyle, alcènyle, alcoxy, alcoxyalkyle, alkylcarbonyle, alkylcarbonylalkyle, alcoxycarbonyle ou alcoxycarbonylalkyle ou

$R^1$ et $R^2$, pris avec la double liaison C=C peuvent représenter un noyau comportant une ou plusieurs insaturations, éventuellement substitué une ou plusieurs fois par un groupe alkyle ayant 1 à 4 atomes de carbone, par un halogène et un groupe hydroxyle, et pouvant aussi contenir des hétéroatomes, ou

$R^2$ et $R^3$, pris avec l'atome de carbone qui les réunit, peuvent représenter un noyau saturé ou insaturé, éventuellement substitué une ou plusieurs fois par un groupe alkyle ayant 1 à 4 atomes de carbone, par de l'halogène et un groupe hydroxyle, le noyau pouvant aussi contenir des hétéroatomes,

$X^1$, $X^2$ et $X^3$, indépendamment l'un de l'autre, représentent un atome d'hydrogène, un groupe alkyle ou un atome d'halogène,

Y représente un groupe furyle, tétrahydrofuryle, thiényle, tétrahydrothiényle; un noyau phényle éventuellement substitué par de l'halogène, par un groupe alkyle, alcoxy et alkylthio ayant chaque fois 1 à 4 atomes de carbone, halogénoalkyle, halogénoalcoxy et halogénoalkylthio ayant chaque fois 1 à 4 atomes de carbone et 1 à 5 atomes d'halogène identiques ou différents, nitro, cyano ainsi que par un groupe phényle éventuellement substitué par de l'halogène et par un groupe alkyle ayant 1 à 4 atomes de carbone; un groupe isoxazolyle éventuellement substitué par un groupe alkyle; 1,2,4-triazol-1-yle, imidazol-1-yle et pyrazol-1-yle, alkyle, alcényle et alcynyle éventuellement substitué par un groupe cyano ou thiocyano; cycloalkyle ou cycloalcényle (éventuellement substitué par un groupe alkyle ayant 1 à 4 atomes de carbone, alcényle ayant 2 à 4 atomes de carbone, halogénoalkyle ayant 1 à 4 atomes de carbone et jusqu'à 5 atomes d'halogène identiques ou différents, halogénoalkyle ayant 2 à 4 atomes de carbone et jusqu'à 5 atomes d'halogène identiques ou différents, par de l'halogène, par un groupe alcoxy ayant 1 à 4 atomes de carbone ainsi que par un pont méthylénique comportant 2 à 5 chaînons); un groupe dihalogénoalkyle; ainsi que les groupements

$-CH_2Az$ $-CH_2R^9$ $-CH_2OR^8$ $-CH_2OR^9$ $-CH_2SR^8$ $-CH_2SR^9$

$-CH_2-SO_nR^8$ $-CH_2SO_n-R^9$ $-CH_2OSOR^8$ $-CH_2OSOR^9$ $-CH_2OSO_2R^8$

$-CH_2OSO_2R^9$ $-CH_2OCOR^8$ $-CH_2OCOR^9$ $-OR^8$ $-OR^9$ $-SR^8$

$-SR^9$ $-NR^{10}R^{11}$ $-CH_2-O-$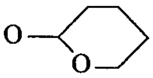

ou

$R^8$ représente un groupe alkyle, alcényle, alcynyle et alcoxyalkyle éventuellement substitués par de l'halogène, par un groupe cyano ou thiocyano;

$R^9$ représente un noyau phényle éventuellement substitué par de l'halogène, par un groupe alkyle, alcoxy, alkylthio ayant chacun 1 à 4 atomes de carbone, halogénoalkyle, halogénoalcoxy et halogénoalkylthio ayant chacun 1 à 5 atomes de carbone et 1 à 5 atomes d'halogène identiques ou différents, nitro, cyano ainsi que par un noyau phényle (éventuellement substitué par de l'halogène et par un groupe alkyle ayant 1 à 4 atomes de carbone),

$R^{10}$ et $R^{11}$ sont identiques ou différents et représentent chacun un atome d'hydrogène, un groupe alkyle, alcényle ou phényle (éventuellement substitué par de l'halogène, par un groupe alkyle, alcoxy et alkylthio ayant chacun 1 à 4 atomes de carbone, halogénoalkyle, halogénoalcoxy et halogénoalkylthio ayant chacun 1 à 4 atomes de carbone et 1 à 5 atomes d'halogène identiques ou différents, nitro, cyano, ainsi qu'un groupe phényle éventuellement substitué par de l'halogène et par un groupe alkyle ayant 1 à 4 atomes de carbone),

Az représente un groupe pyrazol-1-yle, 1,2,4-triazol-1-yle et imidazol-1-yle,

n vaut 1 ou 2,

procédé caractérisé en ce que:

a) on fait réagir des azines de formule:

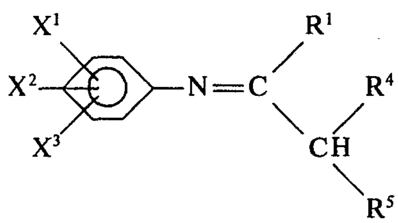

(II)

dans laquelle

$R^1$, $X^1$, $X^2$ et $X^3$ ont le sens indiqué ci-dessus, et

$R^4$ représente un atome d'hydrogène, un groupe alkyle, alcoxy, alcoxyalkyle, hydroxyalkyle ou benzyle; et

$R^5$ représente un atome d'hydrogène, un groupe alkyle, alcényle, alcoxy, alcoxyalkyle, alkylcarbonyle, alkylcarbonylalkyle, alcoxycarbonyle ou alcoxycarbonylalkyle, ou bien

$R^1$ et $R^4$, pris avec la liaison C—C qui les réunit, représentent un noyau saturé ou insaturé, éventuellement substitué une ou plusieurs fois par un groupe alkyle ayant 1 à 4 atomes de carbone, par de l'halogène et un groupe hydroxyle, ou bien

$R^4$ et $R^5$, pris avec l'atome de carbone qui les relie, représentent un noyau saturé ou insaturé, éventuellement substitué une ou plusieurs fois par un groupe alkyle ayant 1 à 4 atomes de carbone, par de l'halogène et un groupe hydroxyle, ce noyau pouvant également contenir des hétéroatomes,

avec les dérivés d'acide de formule (III)

$$Hal-CO-Y \qquad (III)$$

dans laquelle

Hal représente du chlore ou du brome et

Y a le sens indiqué ci-dessus, en opérant éventuellement en présence d'un diluant, et, sans isolement préalable, on soumet les acétanilides résultants, de formule (IV)

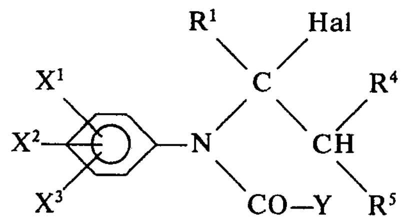

(IV)

dans laquelle

Hal, $R^1$, $R^4$, $R^5$, $X^1$, $X^2$, $X^3$ et Y ont le sens indiqué ci-dessus,

à une élimination d'hydracide halogéné, en opérant éventuellement en présence d'une base, ou bien

b) on fait réagir des chlorures d'acides carbamiques de formule (V):

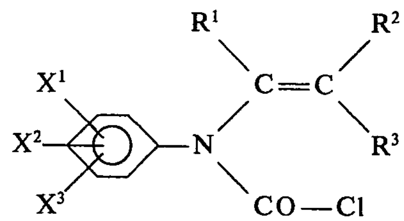

(V)

dans laquelle

$R^1$, $R^2$, $R^3$, $X^1$, $X^2$ et $X^3$ ont le sens indiqué ci-dessus,

avec des composés de formule (VI):

B – Y' (VI),

dans laquelle

Y' représente Az ainsi que les groupements $-OR^8$, $-OR^9$, $-SR^8$, $-SR^9$ et $-NR^{10}R^{11}$, où Az, $R^8$, $R^9$, $R^{10}$ et $R^{11}$ ont le sens indiqué ci-dessus, et

B représente un atome d'hydrogène ou un métal alcalin,

en opérant éventuellement en présence d'un diluant et éventuellement en présence d'un agent de fixation des acides, ou bien

c) on fait réagir des chloracétamides de formule (VII):

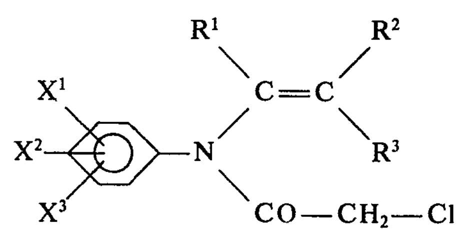

(VII)

dans laquelle

$R^1$, $R^2$, $R^3$, $X^1$, $X^2$, et $X^3$ ont le sens indiqué ci-dessus,

avec des composés de formule (VIII)

B – Y'' (VIII),

dans laquelle

Y'' représente Az ainsi que les groupements $-OR^8$, $-OR^9$, $-SR^8$, $-SR^9$, $-OCOR^8$, $-OCOR^9$, $-OSOR^8$, $-OSOR^9$, $OSO_2R^8$, $-OSO_2R^9$, où
Az, B, $R^8$ et $R^9$ ont le sens indiqué ci-dessus,

en opérant éventuellement en présence d'un diluant et éventuellement en présence d'un agent de fixation des acides, ou bien

d) on fait réagir des hydroxyacétanilides de formule (IX):

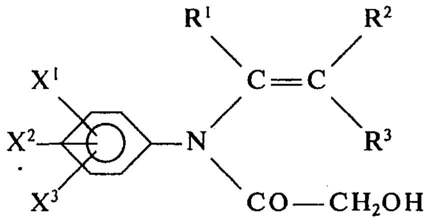

(IX)

dans laquelle

$R^1$, $R^2$, $R^3$, $X^1$, $X^2$ et $X^3$ ont le sens indiqué ci-dessus,

éventuellement après activation à l'aide d'un métal alcalin, avec des halogénures de formule (X):

Hal—Y''' (X),

dans laquelle

Y''' représente $R^8$ ou les groupements $-SO_2R^8$ et $-SO_2R^9$, où $R^8$ et $R^9$ ont le sens indiqué ci-dessus, et
Hal représente le chlore ou le brome,

en opérant en présence d'un diluant et éventuellement en présence d'un agent de fixation des acides, ou bien

e) on oxyde selon des méthodes connues, de façon usuelle, des acétanilides de l'invention, de formule (XI):

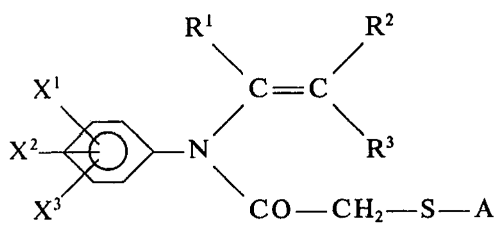

(XI)

dans laquelle

A représente $R^8$ ou $R^9$, et
$R^1$, $R^2$, $R^3$, $R^8$, $R^9$, $X^1$, $X^2$ et $X^3$ ont le sens indiqué ci-dessus.

5. 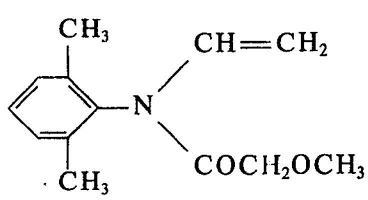

6. 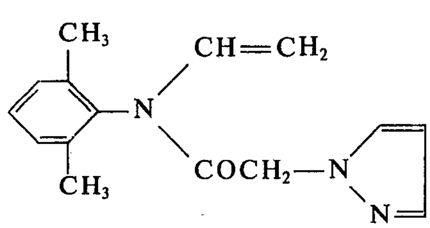

7. Produits fongicides, caractérisé par une teneur en au moins un N-(alcényl)-anilide d'acide carboxylique de formule (I).

8. Procédé pour combattre les champignons, caractérisé en ce qu'on fait agir un N-(1-alcényl)-anilide d'acide carboxylique de formule (I) sur des champignons ou sur leur espace vital.

9. Utilisation des N-(1-alcényl) anilides d'acides carboxyliques de formule (I) pour combattre les champignons.

10. Procédé de préparation de produits fongicides, caractérisé en ce qu'on mélange un N-(1-alcényl)-anilide d'acide carboxylique de formule (I) avec des agents diluants et/ou des produits tensioactifs.